# EUROPEAN PATENT SPECIFICATION

(11) **EP 2 829 221 B1**
(45) Date of publication and mention of the grant of the patent: **24.06.2020**
(21) Application number: 13763733.6
(22) Date of filing: 13.03.2013
(51) Int. Cl.: A61B 3/113, A61B 3/10, A61B 10/00, A61B 5/11, A61B 5/00

(54) **ASPERGER'S DIAGNOSIS ASSISTANCE DEVICE**
VORRICHTUNG ZUR UNTERSTÜTZUNG VON ASPERGER-DIAGNOSEN
DISPOSITIF D'ASSISTANCE AU DIAGNOSTIC DU SYNDROME D'ASPERGER

(30) Priority: 21.03.2012 JP 2012063237; 20.02.2013 JP 2013030686
(43) Date of publication of application: 28.01.2015
(73) Proprietor: National University Corporation Hamamatsu University School of Medicine, Hamamatsu-shi, Shizuoka 431-3192 (JP)
(72) Inventor: MORI Norio, Hamamatsu-shi Shizuoka 431-3192 (JP); SUZUKI Katsuaki, Hamamatsu-shi Shizuoka 431-3192 (JP); TSUCHIYA Kenji, Hamamatsu-shi Shizuoka 431-3192 (JP); SHIMMURA Chie, Hamamatsu-shi Shizuoka 431-3192 (JP); SAKURAI Hirohisa, Shizuoka-shi Shizuoka 424-0911 (JP); TOKUTANI Keijyu, Shizuoka-shi Shizuoka 424-0911 (JP)
(74) Representative: Müller-Boré & Partner Patentanwälte PartG mbB
(86) International application number: PCT/JP2013/056919
(87) International publication number: WO 2013/141098

(56) References cited:
- JP-A- 2011 206 542
- JP-A- 2013 052 116
- US-A1- 2009 097 757
- US-A1- 2011 242 486
- UTTAMA LAHIRI ET AL: "Dynamic gaze measurement with adaptive response technology in Virtual Reality based social communication for autism", VIRTUAL REHABILITATION (ICVR), 2011 INTERNATIONAL CONFERENCE ON, IEEE, 27 June 2011 (2011-06-27), pages 1-8, XP032008769, DOI: 10.1109/ICVR.2011.5971840 ISBN: 978-1-61284-475-6
- JAMES C MCPARTLAND ET AL: "Patterns of Visual Attention to Faces and Objects in Autism Spectrum Disorder", JOURNAL OF AUTISM AND DEVELOPMENTAL DISORDERS, KLUWER ACADEMIC PUBLISHERS-PLENUM PUBLISHERS, NE, vol. 41, no. 2, 25 May 2010 (2010-05-25), pages 148-157, XP019874745, ISSN: 1573-3432, DOI: 10.1007/S10803-010-1033-8
- MARTINEAU J ET AL: "Emotional faces, avatars and objects: Visual fixation patterns in children with Autism Spectrum Disorder (ASD)", INTERNATIONAL JOURNAL OF PSYCHOPHYSIOLOGY, ELSEVIER, AMSTERDAM, NL, vol. 77, no. 3, 1 September 2010 (2010-09-01), page 233, XP027196363, ISSN: 0167-8760 [retrieved on 2010-08-06]
- KEISUKE MATSUMURA ET AL.: 'Precise and Real- time Pupil Position Estimation in Color Camera Face Images Based on Near-infrared Pupil Detection Method' THE JOURNAL OF THE INSTITUTE OF IMAGE INFORMATION AND TELEVISION ENGINEERS vol. 65, no. 12, 2011, pages 1783 - 1787, XP055160955

## Description

### TECHNICAL FIELD

The present invention relates to a device for supporting diagnosis of autism. More particularly, the present invention relates to a device for supporting diagnosis of autism using an eye-gaze detection technique, each supporting early definite diagnosis of patients of autism.

Hereinafter, the term "autism" is used as a generic term including autism-related diseases, such as Asperger syndrome and Asperger disorder (pervasive development disorders with abnormality in sociality, interest and communication), and the present invention can be applied also to these autism-related diseases.

### BACKGROUND ART

Autism is one of the development disorders with impairment in sociality (prevalence is supposedly 1%). Incorrect diagnosis or detection of autism of a child may result in serious trouble in daily life and school life. Furthermore, lowering of self-esteem and development of psychiatric symptoms, such as anxiety and dysphoria, are expected to occur.

However, established effective medication of autism has not been found yet. Therefore, the only way to improve prognosis of autism is early diagnosis and early (from the age younger than 3 years old) educational intervention based on the diagnosis.

Unfortunately, reliable early diagnosis of autism is difficult to achieve with current standard clinical technique. For example, in conventional diagnosis of autism, evaluation and diagnosis are conducted by a pediatrician or child psychiatrist based on behavior of infants. Unfortunately, there are not so many experts (specialists) and early diagnosis is difficult. Furthermore, objective evaluation is currently difficult since the evaluation result varies depending on evaluators.

In current diagnosis of autism by specialists, experiential determination is made in interview based on appearance, or components of collected blood are examined. However, there is a problem in that the appearance-based determination requires a great deal of experience of specialists and is difficult to quantify. The blood test requires cumbersome procedure of collecting blood. Furthermore, these examination means are currently neither effective nor reliable in the examination of infant. This is because it is almost impossible to communicate with an infant in an interview and application of the determination based on blood components to an infant younger than 3 years old is still in the laboratory stage. Furthermore, there is a serious problem of failure or delay in medical consultation that occurs when parents of an infant do not realize that fact that the infant has autism.

In view of current situation above, with respect to autism of not only adults but also children, especially infants, a method, device, and support system which enable early detection and early definite diagnosis by experts (specialists) based on objective evaluation have been called for.

In recent years, it has become more certain that abnormality is found in the distribution of points of regard of undiagnosed infants with autism. Specifically, it is becoming clear that an infant with autism has a feature that he/she cannot correctly focus on an eye gaze of another person. This abnormality is regarded as derived from the essence of autism, impairment in sociality. Furthermore, this abnormality is regarded as a symptom appearing in an extremely early stage.

By the use of a preferable eye-gaze detection technique, this abnormality can be correctly detected and utilized as an objective index for early diagnosis of autism. Based on this idea, the present inventors focused on this abnormality.

To obtain such abnormality in the distribution of points of regard, as a conventional eye-gaze detection technique (referred to as "eye-gaze detection technique", "eye-gaze sensing technique", "eye-gaze detection input technique", "eye-gaze detection recognition technique", "eye-gaze detection tracing technique", and the like), for example, a method for detecting eye gaze of a subject, uses: a first imaging camera for measuring the position of a pupil relative to a coordinate system; a second imaging camera having a light source arranged at a known position in the coordinate system and forming a corneal reflection center to obtain data of a distance r from the corneal reflection center to a pupil center and an angle ϕ of distance r relative to a coordinate axis of the coordinate system; and calculation means for calculating the direction of eye gaze based on information from each of the cameras . Further, some techniques using this method (device, technique and the like for detecting eye gaze) are also proposed (for example, see Patent Literature 1 to 5).

Patent Literature 6 discloses, as another technique similar to the "eye-gaze detection technique" of above-mentioned patent documents, an eye detection installation comprising:
one or more light sources for emitting light in directions toward the head of a user,
a detector for receiving light from the head of a user and to repeatedly capture pictures thereof, and
an evaluation unit connected to the detector for determining the position and/or gaze direction of an eye,
wherein the evaluation unit is arranged to determine, in a picture captured by the detector, an area in which an image of an eye or images of eyes is/are located and, after determining the area, to control the detector to forward to the evaluation unit only information about successive or following pictures that corresponds to the determined area of the image captured by the detector.

Patent Literature 7 discloses a device for recognizing eye gaze in which:
an eye that is subjected to gaze recognition is irradiated with light,
an image is formed by an imaging camera, the image having 3 or more characteristic points on the cornea of the eye,
the center of curvature of the cornea of the eye is determined from the characteristic points of the cornea on the formed image, and
the eye-gaze direction is recognized from the information of the center of curvature of the cornea and position of the center of the pupil,
the device comprising:
   provisional eye-gaze direction calculating means which calculates a provisional eye-gaze direction from the positional relationship between the center of curvature of the cornea and the center of the pupil; and
   corneal area determining means which determines a restricted corneal area from the provisional eye-gaze direction and the information of the position of the pupil,
   treating means which, when the characteristic points on the image are present within the restricted corneal area, regards the provisional eye-gaze direction as the result of the recognition of eye gaze and, when a portion of the characteristic points on the image is not present within the restricted corneal area, selects additional characteristic points on the image present within the restricted corneal area, determines additional center of curvature of the cornea of the eye from the additional characteristic points on the image selected, recognizes additional eye-gaze direction from the information of the additional center of curvature of the cornea and position of the center of the pupil, and regards the additional eye-gaze direction as the result of the recognition of eye gaze.

Patent Literature 8 discloses an eye tracking system for monitoring the movement of a user's eye, the system comprising:
(a) video data input means for receiving video data produced by eye imaging means (imaging camera) monitoring the user's eye;
(b) spot location means for determining, from the video data, the location of a reference spot formed on the user's eye by illumination of the user's eye by a point source of light, the spot location means including adaptive threshold means for providing an indication of parts of the image produced by the eye imaging means which have a brightness greater than a threshold value, and spot identification means for selecting a valid reference spot by comparing the parts of the image with predetermined validity criteria;
(c) pupil location means for determining, from the video data, the location of the centre of the pupil of the user's eye relative to the reference spot in order to determine the user's line of gaze,
   the pupil location means comprising:
   selection means which selects a pupil tracking window comprising a portion of the image produced by the eye imaging means, the portion corresponding to the location of the valid reference spot;
   edge selecting means which selects the edge of the pupil by selection of those parts of the gradient of the image portion in the pupil tracking window which have a gradient greater than a threshold value; and
   centre determining means which determines the centre of the pupil by referring to the points selected for the edge of the pupil;
   the centre determining means comprising:
      triad selection means for substantially randomly selecting three superthreshold pixels to form a triad for further processing, from among a plurality of pixels of pupil image data; and
      triad processing means for determining the centre and radius of a hypothetical circle passing through each of the selected pixels; and (d) display means for indicating the user's point of regard from the user's line of gaze determined by the pupil and spot location means.

Patent Literature 9 to 11 disclose techniques studied for achieving hands-free, eye-gaze conducted operation and control instruction for device. Specifically, an eye-gaze detecting unit 2 uses an electrode for detecting the movement of the eye of the user who is wearing the eye-gaze detecting unit 2 on his or her head, instead of using the camera (eye imaging means) for capturing an image of the eye. The electrode is attached to a portion, of an electronic device mainly using an earphone or a headphone, to be in contact with a portion around an ear.

However, prior art in which the above-mentioned "eye-gaze detection technique" and the like using at least the camera or the electrode is applied to support diagnosis of autism is rarely found. Such rare examples include Patent Literature 12 and 13 filed by the present inventors in the course of developing development subject: POINTS OF REGARD DETECTION DEVICE FOR DIAGNOSING INFANT WITH AUTISM in the entrusted research and development related to R&D program (Development of Systems and Technologies for Advanced Measurement and Analysis). Other relevant prior art includes "Dynamic gaze measurement with adaptive response technology in Virtual Reality based social communication for autism" from Lahiri et al, Proc. 2011 Int. Conf. on Virtual Rehabilitation (ICVR). US 2011/0242486 A1 discloses a system to support autism diagnosis based on a state of a subject looking at a target, including an eye-gaze point detection unit. "Patterns of Visual Attention to Faces and Objects in Autism Spectrum Disorder" from McPartland et al. in Journal of Autism and Developmental Disorders Vol. 41 pp. 148-157 discloses a number of visual patterns to help diagnose autism.

As described later, the present invention can use "eye-gaze detection technique" as described in Patent Literature 1 to 11. Of the techniques, those described in the above-mentioned Patent Literature 1 to 5 are suitable for an infant who has small pupils, and cannot understand what is spoken and thus might not stand still as instructed.

Patent Literature 1: Japanese Patent No. 4517049
Patent Literature 2: Japanese Patent No. 4452835
Patent Literature 3: Japanese Patent No. 4452836
Patent Literature 4: Japanese Patent No. 4491604
Patent Literature 5: Japanese Patent No. 4528980
Patent Literature 6: Japanese Patent No. 4783018
Patent Literature 7: Japanese Patent No. 3453911
Patent Literature 8: Japanese Patent No. 4181037
Patent Literature 9: Japanese Patent Application Laid-open No. 2006-340986
Patent Literature 10: WO2009/142008
Patent Literature 11: Japanese Patent Application Laid-open No. 2011-120887
Patent Literature 12: Japanese Patent Application Laid-open No. 2011-206542
Patent Literature 13: Japanese Patent Application No. 2011-192387 (specification)

### DISCLOSURE OF THE INVENTION

The invention is defined in independent claim 1. It is an object of the present invention to provide, in view of the above-mentioned problems, a device for supporting diagnosis of autism which can provide support for early detection and early definite diagnosis of autism (especially in infants) based on objective evaluation, using a conventionally proposed "eye-gaze detection technique".

In the autism diagnosis support, detection of the eye-gaze movement of the subject with a high accuracy is nothing more than a prerequisite. The support has to be based on the important concept of "extracting a subject who might have autism without fail". In other words, the support for the autism diagnosis carried out by a doctor is required to be highly sensitive and specific.

Assume a case where 10 individuals with autism are in a total of 1000 subjects. Here, it is important that the screening carried out detects the 10 individuals with autism without fail, even though a few typically developing individuals, in the subjects, might be determined as "autism suspected" in the process.

All things considered, the main object of the present invention is to provide diagnosis support that can achieve the screening with which the individuals with autism are detected without fail. Preferably, the screening involves the minimum possible number of the typically developing individuals determines as "autism suspected" as described above.

The present inventors, who have proposed the inventions disclosed in Patent Literature 12 and 13, have found a way to achieve the main object in the autism diagnosis support described above, with an advanced screening. Specifically, such a screening is achieved by evaluating a tendency of the individuals with autism to fail to correctly focus on an eye gaze of another person, while also taking other tendencies of the eye gaze unique to individuals with autism into consideration.

The term "contribute to the evaluation (or evaluate)" corresponds to detection of the symptom derived from the essence of autism, impairment in sociality, from the eye-gaze movement to suggest an objective index for early diagnosis of autism, and does not correspond to actual medical practice (definite diagnosis).

The present disclosure provides a diagnosis support method with improved convenience. For example, an examination itself for data detection only can be conducted, even in an area with a small number of experts (specialists) or in a case with no expert (specialist) present (such as a group examination in a school or local health center) . The definite diagnosis can be made by an expert (specialist), later or immediately for early detection, based on the evaluation result of the detected data, even in a distant place using communicating means. Furthermore, a doctor of another medical field or the like can make recommendation to obtain definite diagnosis by an expert (specialist) based on the evaluation result of the detected data.

The present inventors have made extensive and intensive studies to solve the above-mentioned problems. As a result, it has been found that the difference in a tendency of an eye-gaze movement between typically developing (healthy) children (hereinbelow referred to as "typically developing individuals" including adults, children and infants) and children with autism (hereinbelow referred to as "individuals with autism" including adults, children and infants) as subjects can be detected using the above-mentioned "eye-gaze detection technique" by applying the "eye-gaze detection technique", such as those mentioned in the above-mentioned prior art documents, to the above-mentioned abnormality in the distribution of points of regard of infants with autism (which is regarded as derived from the essence of autism, impairment in sociality, and also regarded as a symptom appearing in an extremely early stage) as a technique for correctly detecting this abnormality and, in conjunction with this technique, displaying a predetermined "combination image", with a predetermined configuration, to the subjects. The present invention has been completed, based on this novel finding.

Specifically, a first aspect of the present disclosure provides a method for supporting autism diagnosis for a subject, using an eye-gaze detecting unit (A) at least including a camera portion (a1) capturing an image of an eye of the subject, or an electrode portion (a2) to be mounted on a head of the subject and detecting a movement of the eye, or a display portion (a3) to be disposed at a position in an eye gaze direction of the subject, the method including: displaying, on a screen of the display portion (a3), a combination image for sequentially displaying at least two images including a predetermined human image (I) and a predetermined non-human image (II); and evaluating an eye-gaze position of the subject by detecting eye-gaze position information on the subject looking at the combination image in use of the eye-gaze detecting unit (A), then inputting the eye-gaze position information on the subject in an eye-gaze position information storing portion, and comparing, based on an eye-gaze position evaluation algorithm, the eye-gaze position information on the subject with eye-gaze position information on an individual with autism and/or a typically developing individual.

A second aspect of the present disclosure provides the autism diagnosis support method of the first aspect, in which in the eye-gaze position evaluation algorithm, the predetermined human image (I) includes a still image (i) and a moving image (ii) partially moving, and a frequency of an eye-gaze movement, in a case where the still image (i) or the moving image (ii) partially moving is displayed on the screen of the display portion (a3), is worked out on the basis of a contrast or difference between the typically developing individual and the individual with autism in terms of a tendency of an eye-gaze movement where the frequency of the eye-gaze movement of the typically developing individual to a moving portion of the moving image is high but that of the individual with autism is low.

A third aspect of the present disclosure provides the autism diagnosis support method of the second aspect, in which the predetermined human image (I) includes three types of images, which are a still image (ia) of a face, a moving image (iia) of the face where only an eye is opened and closed, and a moving image (iib) of the face where only a mouth is opened and closed.

A fourth aspect of the present disclosure provides the autism diagnosis support method of the third aspect, in which the frequency of the eye-gaze movement is based on a contrast or difference between the typically developing individual and the individual with autism in terms of a tendency of an eye-gaze movement where, while the moving image (iia) of the face where only the eye is opened and closed is displayed, the frequency of the eye-gaze movement of the typically developing individual to a periphery of the eye is high but that of the individual with autism is low.

A fifth aspect of the present disclosure provides the autism diagnosis support method of the third aspect, in which the frequency of the eye-gaze movement is based on a contrast or difference between the typically developing individual and the individual with autism in terms of a tendency of an eye-gaze movement where the frequency of the eye-gaze movement of the individual with autism to (iia) in a case where the moving image (iib) of the face where only the mouth is opened and closed is first displayed and then the moving image (iia) of the face where only the eye is opened and closed is displayed is low compared with that of the typically developing individual in a case where the still image (ia) of the face or the moving image (iib) of the face where only the mouth is opened and closed is displayed.

A sixth aspect of the present disclosure provides the autism diagnosis support method of any one of the first to the third aspects, in which an image of a person whom the subject knows is used as the predetermined human image (I).

A seventh aspect of the present disclosure provides the autism diagnosis support method of the second aspect, in which in the eye-gaze position evaluation algorithm, the predetermined non-human image (II) includes at least one type selected from an appearance prediction image (α), an illusion recognition image (β), and a difference search image (γ).

An eighth aspect of the present disclosure provides the autism diagnosis support method of the first aspect, in which
the appearance prediction image (α) of the predetermined non-human image (ii) is a moving image formed of a moving body image (α1), or optionally formed as a combination of the moving body image (α1) and a hiding body image (α2), and
the frequency of the eye-gaze movement at a time of redisplaying of the moving body image (α1) at a predetermined position in the display portion (a3), after first displaying the moving body image (α1) in such a manner as to move on the screen on the display portion (a3) and then making the moving body image (α1) transition to a non-displayed state by being off the screen of the display portion (a3) or by the hiding body image (α2), is based on a contrast or difference between the typically developing individual and the individual with autism in terms of a tendency of an eye-gaze movement where the frequency of the eye-gaze movement of the typically developing individual to a position where the moving body image (α1) is redisplayed is high but that of the individual with autism is low.

A ninth aspect of the present disclosure provides the autism diagnosis support method of the seventh aspect, in which the frequency of the eye-gaze movement at the time of redisplaying is not used for the evaluation of the frequency of the movement when the redisplaying is implemented for a first time, but is used for the evaluation of the frequency of the movement when the redisplaying is implemented for a second time or after, where a movement, under a certain rule, of a moving body image (α1) is predictable.

A tenth aspect of the present disclosure provides the autism diagnosis support method of the seventh aspect, in which
the illusion recognition image (β) of the predetermined non-human image (II) is an image formed of pictures including an illusion causing element (β1) and a non-illusion causing element (β2), and
the frequency of the eye-gaze movement, in a case where the illusion causing element (β1) is displayed, is based on a contrast or difference between the typically developing individual and the individual with autism in terms of a tendency of an eye-gaze movement where the frequency of the eye-gaze movement of the typically developing individual between a position where the illusion causing element (β1) is displayed and a position where the non-illusion causing element (β2) is displayed is high, but that of the individual with autism is low.

An eleventh aspect of the present disclosure provides the autism diagnosis support method of the seventh aspect, in which the difference search image (γ) of the predetermined non-human image (II) is an image formed of a combination of a plurality of identical pictures (γ1) having the same or similar appearance, and one or several different pictures (γ2) having a shape different from those of the identical pictures, and
the frequency of the eye-gaze movement, in a case where the identical pictures (γ1) and the different pictures (γ2) are displayed in a mixed manner on the display portion (a3), is based on a contrast or difference between the typically developing individual and the individual with autism in terms of a tendency of an eye-gaze movement where the frequency of the eye-gaze movement of the typically developing individual between a position where the identical picture (γ1) is displayed and a position where the different picture (γ2) is displayed is low, but that of the individual with autism is high.

A twelfth aspect of the present disclosure provides the autism diagnosis support method of the first aspect, wherein before the combination image is displayed on the screen of the display portion (a3), a preliminary image leading image (θ) is displayed on a display member to lead the eye gaze of the subject to a predetermined position in advance.

A thirteenth aspect of the present disclosure provides the autism diagnosis support method in claim 1 of the second aspect, in which
in the evaluation of the frequency of the eye-gaze movement of the typically developing individual and the individual with autism, the frequency is detected under a condition where whether the frequency of the movement obtained from the detected eye-gaze position information on the subject is high or low depends on an average time from a time at which each image is displayed on the screen of the display portion.

A fourteenth aspect of the present disclosure provides the autism diagnosis support method of the second aspect, in which
the eye-gaze position evaluation algorithm sets a threshold value for the frequency of the eye-gaze movement based on a database having stored therein previously obtained eye-gaze position information on the subject and definite diagnosis of the subject as to whether the subject is an individual with autism.

A fifteenth aspect of the present disclosure provides an autism diagnosis support system including:
(a) eye-gaze detecting means using an eye-gaze detecting unit (A) at least including a camera portion (a1) capturing an image of an eye of a subject, or an electrode portion (a2) to be mounted on a head of the subject and detect a movement of the eye, or a display portion (a3) to be disposed at a position in an eye-gaze direction of the subject, in order to detect eye-gaze position information on the subject looking at a screen of the display portion;
(b) means for inputting the eye-gaze position information on the subject;
(c) eye-gaze evaluation means for evaluating an eye-gaze position of the subject with an eye-gaze position evaluation algorithm based on position information in a case where the eye-gaze position information on the subject is displayed on the screen of the display portion (a3), as a combination image for sequentially displaying at least two images including a predetermined human image (I) and a predetermined non-human image (II), the eye-gaze position evaluation algorithm comparing the eye-gaze position information on the subject with eye-gaze position information on an individual with autism and/or a typically developing individual; and
(d) display means for displaying an evaluation result of the eye-gaze position of the subject.

A sixteenth aspect of the present disclosure provides an autism diagnosis support device supporting autism diagnosis by using a combination image for sequentially displaying at least two images including a predetermined human image (I) and a predetermined non-human image (II), the device including:
(i) an eye-gaze detecting portion using eye-gaze detecting means to detect eye-gaze position information on a subj ect looking at the combination image displayed in an eye-gaze direction of the subject;
(ii) an eye-gaze position information storing portion storing the eye-gaze position information detected by the eye-gaze detecting portion;
(iii) an eye-gaze position information displaying unit displaying the eye-gaze position information on the subject stored in the eye-gaze position information storing portion;
(iv) an eye-gaze position information evaluating portion evaluating the eye-gaze position information on the subject displayed on the eye-gaze position information displaying portion through comparison with eye-gaze position information on an individual with autism and/or a typically developing individual, on the basis of an eye-gaze position evaluation algorithm comparing the eye-gaze position information on the subject with the eye-gaze position information on the individual with autism and/or the typically developing individual;
(v) an evaluation result outputting portion outputting an evaluation result obtained by the eye-gaze position information evaluating portion; and
(vi) an evaluation result storing portion storing the evaluation result output from the evaluation result outputting portion or the evaluation result obtained by the eye-gaze position information evaluating portion.

The object can be achieved with the configurations of the aspects.

First of all, in the first, the fifteenth, and the sixteenth aspects of the present disclosure "eye-gaze detection technique" as disclosed in the above-mentioned Patent Literature are applied. Furthermore, a specific "combination image with at least two sequential images" in a predetermined configuration is displayed to the subject, in conjunction with the technique. Thus, the support can be provided to early definite diagnosis for a patient with autism, based on the concept of the screening of detecting the individuals with autism without fail. The technique also allows someone who is not a specialist to show how much the patient is likely to have autism, and suggest whether the diagnosis is required. The technique has a particular advantage that support can be provided for early detection and early definite diagnosis of autism, even in an infant before the age that the identification by a specialist as an individual with autism can be applied to, based on objective evaluation.

In the second aspect of the present disclosure, a predetermined moving image is displayed to the subject. Thus, the tendency difference between the typically developing individual and the individual with autism regarding the moving portion on the screen is extracted and contribute to the evaluation.

In the third aspect of the present disclosure, a predetermined moving image is displayed to the subject. Thus, the tendency difference between the typically developing individual and the individual with autism in the frequency of the eye-gaze movement is extracted and can contribute to the evaluation.

In the fourth aspect of the present disclosure, the moving image where the mouth of the face image is opened and closed is displayed to the subject. Thus, the tendencies of the frequency of the eye-gaze movement of the typically developing individual and the individual with autism can more contribute to the evaluation.

In the fifth aspect of the present disclosure, the specific face images are displayed to the subject in a predetermined order (first mouth and then eyes are opened and closed). Thus, the tendencies of the frequency of the eye-gaze movement of the typically developing individual and the individual with autism can more contribute to the evaluation.

In the sixth aspect of the present disclosure, the image of the face familiar to the subject (familiar face) is used. Thus, a state where the subject can more easily look directly in the eyes of the displayed face image is achieved to increase the tendency of the typically developing individual to look directly in the eyes . Thus, the tendency of the eye-gaze movement of the individual with autism that tends not to look into the eyes can more contribute to the evaluation.

In the seventh aspect of the present disclosure, the appearance prediction image, the illusion recognition image, or the difference search image is used as the predetermined non-human image. Thus, not only the tendency of the frequency of the eye-gaze movement in the predetermined human image, but also the tendency difference between the typically developing individual and the individual with autism in the frequency of the eye-gaze movement can be extracted for these images can be extracted to be further taken into consideration to contribute to the evaluation.

In the eight aspect of the present disclosure, the appearance prediction image, the illusion recognition image, or the difference search image is sequentially displayed after the predetermined human image. Thus, the tendency difference between the typically developing individual and the individual with autism in the frequency of the eye-gaze movement can be extracted for these images to be further taken into consideration to contribute to the evaluation.

In the ninth aspect of the present disclosure, the appearance prediction image is used to extract whether the subject is looking at the position where the moving body appears on the screen is extracted. Thus, the tendency of the typically developing individual to look at the moving body in a relatively predicting manner, and the tendency of the individual with autism to not look at the moving body in the relatively predicting manner can contribute to the evaluation.

In the tenth aspect of the present disclosure, in the appearance prediction image, the moving body is repeatedly displayed on the screen based on a predetermined movement pattern, and whether the subject is looking at the position where the moving body reappears on the screen for the second time or after is extracted. Thus, the difference among the subjects in ability is reduced to contribute to the evaluation.

In the eleventh aspect of the present disclosure, the illusion recognition image is displayed to the subject. Thus, the tendency of the typically developing individual to look at the illusion causing portion and the tendency of some individuals with autism to not look at the illusion causing portion can contribute to the evaluation.

In the twelfth aspect of the present disclosure, the illusion recognition image is displayed to the subject. Thus, the tendency of the typically developing individual to look at the illusion causing portion and the tendency of some individuals with autism to not look at the illusion causing portion can contribute to the evaluation.

Furthermore, in the thirteenth aspect of the present disclosure, the difference search image is displayed to the subject Thus, the tendencies of the typically developing individual at the point of trying to find the difference and a point where the difference is found, as well as the tendency of the individual with autism of not trying to find the difference or having an excellent ability to find the difference in a short period of time can contribute to the evaluation.

In the fourteenth aspect of the present disclosure, the leading image (θ) is displayed and thus, the eye-gaze position of the subject can be led to a predetermined position in advance before the next image is displayed. Thus, the evaluation is less likely to be affected by the displacement of the eye-gaze position at the time of displaying. Thus, the tendencies of the movement of the typically developing individual and the individuals with autism, can be stably obtained to contribute to the evaluation

### BRIEF DESCRIPTION OF THE DRAWINGS

Fig. 1 is a figure schematically showing a configuration of a method and system for supporting autism diagnosis.
Fig. 2 is a figure showing examples of a combination image.
Fig. 3 is a figure showing examples of a predetermined human image (I).
Fig. 4 is a figure showing examples of an appearance prediction image (α) as a non-human image (II).
Fig. 5 is a figure showing examples of an illusion recognition image (β) as the non-human image (II).
Fig. 6 is a figure showing examples of a difference search image (γ) as the non-human image (II).
Fig. 7 is a figure showing examples of a leading image (θ).
Fig. 8-1 is a figure showing an example of an eye-gaze position evaluation algorithm.
Fig. 8-2 is a figure showing an example of an eye-gaze position evaluation algorithm.
Fig. 8-3 is a figure showing an example of an eye-gaze position evaluation algorithm.
Fig. 9-1 is a figure showing an example of the eye-gaze position information that is displayed.
Fig. 9-2 is a figure showing an example of the eye-gaze position information that is displayed.
Fig. 10 is a figure showing examples of an evaluation of an eye-gaze position.

### BEST MODE FOR CARRYING OUT THE INVENTION

In the description, first, an eye-gaze detecting unit 2 suitably used in the present invention will be described. Then, an eye-gaze position evaluation algorithm as well as an autism diagnosis support device as a whole, practically equivalent to an autism diagnosis support system, will be described in conjunction with a combination image.

Subsequently, an autism diagnosis support method will be described in conjunction with operation modes of the autism diagnosis support device (in the description below, the autism diagnosis support system and the autism diagnosis support method may be described while being referred to as the autism diagnosis support device for the sake of description).

### EMBODIMENT

First of all, the eye-gaze detecting unit 2 (eye-gaze detection portion) is described. The eye-gaze detecting unit 2 is used as eye-gaze detecting means (a) in the autism diagnosis support system of the present invention.

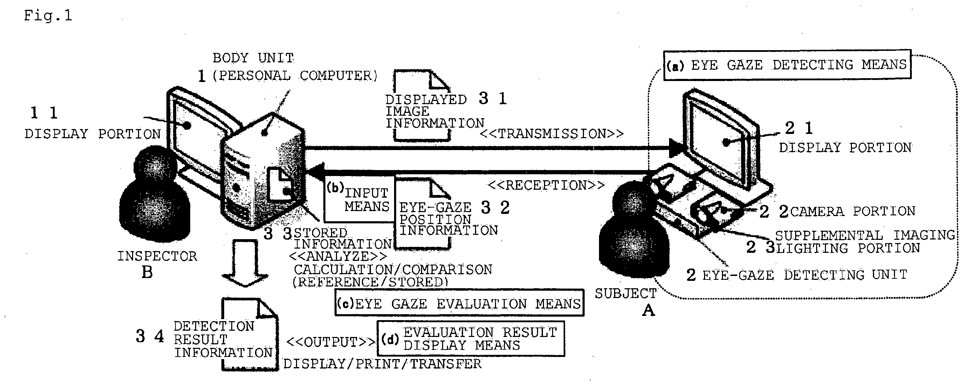

As shown in Fig. 1, the eye-gaze detecting unit 2 includes a camera portion 22 that captures an image of an eye of a subject A and a display portion 21 that displays an image. Optionally, a supplemental imaging lighting portion 23 is provided for ensuring that an image of a pupil is captured without fail. The display portion 21 may be a commercially available display, and is not particularly limited. Specifically, a liquid crystal display, aCRT, aprojector, and the like may be used, and the size and the shape of a display section therein may be set as desired. Although not elaborated in the figure, an electrode (electrode portion) that come into contact with a head, like a headphone or an earphone, to detect the movement of the eye may be used instead of the camera portion 22. The supplemental imaging lighting portion 23 is provided as appropriate in accordance with conditions such as sensitivity of the camera portion 22 and the brightness of the periphery.

The eye-gaze detecting unit 2 detects the pupil of the subject A, and outputs eye-gaze position information 32 representing a position of a target (display portion 21) viewed by the subject A. Specifically, the eye-gaze detecting unit 2 can output the eye-gaze position information 32 indicating where the subject A is looking on the screen of the display portion 21 and when, based on the positional relationship among the subject A, the camera portion 22, and the display portion 21.

Specifically, the eye-gaze position information 32 is not limited to be in the form of one of numeral data and image data, as long as the picture and time of the image to be displayed as described later can be identified without inconsistency with the position and time indicated by the eye-gaze position information.

More specifically, the eye-gaze position information 32 may be output as numeral data indicating relative position coordinates, such as two-dimensional data (X, Y), corresponding to a position on the screen of the display portion 21 as well as the imaging time. Here, the position on the screen of the display portion 21 may be irrelevant. For example, based on the position of the eye of the subject A captured by the camera portion 22 of the eye-gaze detecting unit 2, the eye-gaze position information 32 may be output as numeral data indicating the absolute position coordinate unique to the camera portion 22 and the image capturing time. Alternatively, theeye-gaze position information 32 may be output as the image data instead of the numeral data. Specifically, the eye-gaze position information 32 may be obtained by directly combining a plotted point as the eye-gaze position captured by the camera portion 22 with displayed image data.

Preferably, for example, the eye-gaze position information 32 is output after being converted into position information corresponding to the resolution and dot position information (position information) of the display portion 21 (display) used for the eye-gaze detecting unit 2. In this way, consistency, with respect to the position of the picture displayed on the screen, can be easily ensured. For example, the eye-gaze position information 32 is obtained by converting the eye-gaze position information 32 of the subject A in accordance with the two-dimensional data (X, Y) on the screen of the display portion 21. For example, for the display portion 21 as a display with the screen resolution of 480 (vertical) × 640 (horizontal) dots, the eye-gaze position information 32 of the eye-gaze detecting unit 2 may be adjusted to be output after being converted into position information indicating a position on the screen based on the 480 (vertical) × 640 (horizontal) dots corresponding to the screen resolution.

The eye-gaze position information 32 is not limited to be in the form of two-dimensional data (X, Y) in a planer image displayed on the screen as described above, and may be stereoscopic three-dimensional data (X, Y, Z).

Capability of detecting eye-gaze positions of the subject A with the highest possible accuracy is an important aspect to be a favorable conventional and/or commercially available detecting means. In this context, for example, application of the eye-gaze detection techniques mentioned in the above-mentioned Patent Literature 1 to 5 (including other techniques of any inventions and applications published) to the eye-gaze detecting unit 2 can provide an extremely suitable configuration for cases requiring correction such as those for an infant as a subject moving around ceaselessly, the subject A wearing glasses, curvatures of eyes varying among the subjects A, and the like, which have been considered as difficult to detect the eye gaze. Specifically, the eye-gaze can be detected in such cases with less error, high accuracy and small size of device. Thus, a technique extremely suitable for accurately and easily detecting eye gaze can be achieved.

One typical device of the eye-gaze detecting means has been developed by Shizuoka University [the device published in Collection of Papers for Information Science and technology Forum 9(3), 589-591, 2010-08-20 , "device for detection of points of regard with easy calibration by a stereo camera, which allows head movement"] . In the present invention, a description is given under an assumption that the unit is used so that the eye-gaze position information 32 is adjusted to be converted into the position information on the screen of the display portion 21, to be output.

The eye-gaze detecting unit 2 detects the eye-gaze position information on the subject A, in accordance with the operation of and instruction on the body unit 1 and the display portion 21 as described later. Still, the eye-gaze detecting unit 2 may not necessarily operate in conjunction with the body unit 1 and the display portion 21. In such a case, the eye-gaze position information constantly output from the eye-gaze detecting unit 2 may be received, calculated, and analyzed on the side of the body unit 1. Thus, the position of the displayed pictures on the displayed image information and the displayed eye-gaze position of the subject A are identified.

Next, a combination image used for the displayed image information 31 of the present invention will be described.

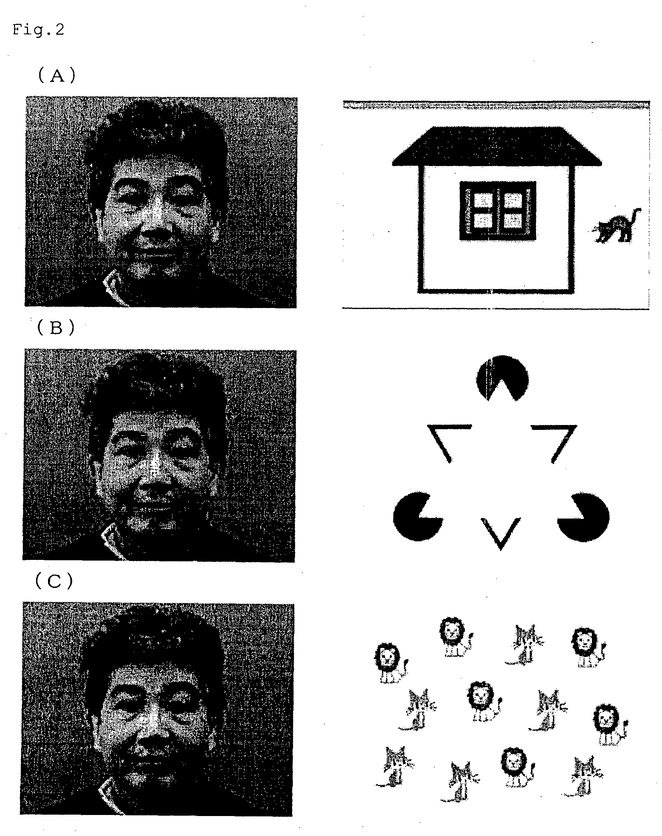

The combination image is the displayed image information 31 for the displaying on the display portion 21 of the eye-gaze detecting unit 2 described above. The combination image is configured in such a manner that a predetermined human image (I) and a predetermined non-human image (II), are sequentially displayed as shown in Figs. 2A to 2C. The combination image includes images each displayed for several seconds to several tens of seconds, and thus is displayed as a whole for several minutes at the longest. The order of (I) and (II) and the number of sequential images are not limited.

The term "sequentially" does not necessarily indicate the consecutive displaying in time series. Therefore, a case where a leading image is displayed in between two images also counts as the combination image, and thus the term should be construed as a sequence in a series of diagnosis supports.

The predetermined human image (I), which is a human image selected from an image of an actual person, an animation character, a humanized animal, and the like, includes (i) a state where the entire image remains still (still image) and (ii) a state involving a certain movement on the screen (moving image partially moving) . The still image (i) is an image where the human image has the body and the facial expression remaining still, and includes the paused state of a moving image. The moving image (ii) is an image where the human image has a part of the body moving, and includes a change in the facial expression.

The predetermined human image (I) may be configured in such a manner that the body is entirely or partially displayed. Preferably, while taking into account the size of the screen of the display portion 21, the image as a moving image including an upper half of the body or an area around the face is displayed. Thus, the subject A checks the movement of the eye, the mouth, and their periphery regarding the facial expression in particular. Preferably, the image is displayed with a size close to the actual size, and with the movement of the eye and the mouth clearly displayed.

Furthermore, an image of a person whom the subject A knows (a familiar face) is preferable, so that the subject A can more easily look directly at the human image (especially in the eye). For example, an image of a person in relation in the first or the second degree of kinship to the subject A may be used.

Such images are not particularly limited and may be an image captured in advance or in real time.

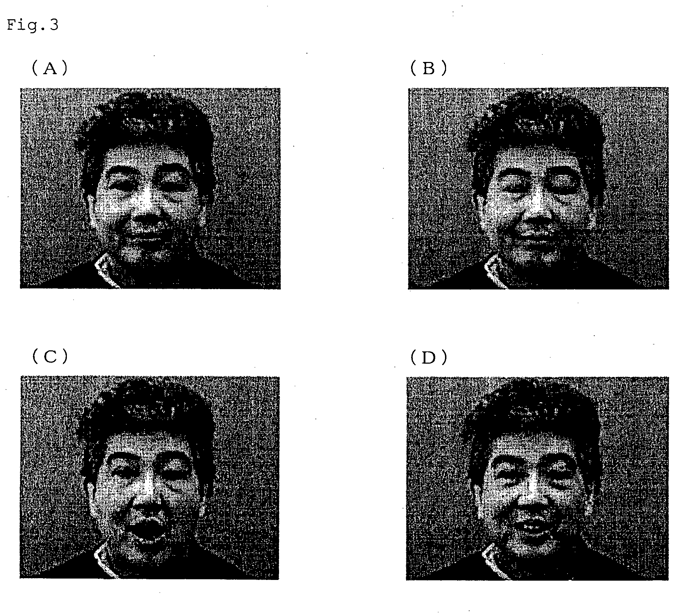

The predetermined human image (I) is an image preferably including images (ia), (iia), and (iib) each displayed for several seconds. As shown in Fig. 3A, the image (ia) is an image of a person with his eyes opened and his mouth closed (face image remaining still as a whole) . As shown in Fig. 3B, the image (iia) is an image of the person repeatedly opening and closing his eyes for several seconds with his mouth closed (face image where only the eyes are opening and closing). As shown in Fig. 3C, the image (iib) is an image of the person repeatedly opening and closing his mouth for several seconds with his eyes opened (face image where only the mouth is opening and closing).

Here, in the display portion 21, for clearly recognizing the opening and closing movements of the eyes, the following important conditions need to be considered for the image (iia). Specifically, the eyes need to be more slowly and firmly opened and closed compared with the normal instantaneous blinking. The blinking needs to be repeated sequentially without stopping the movement of the eye (without rest).

Thus, in the present invention, the normal instantaneous blinking is not regarded as the opening and the closing of the eye. According to Wikipedia, the average normal blinking speed is 0.1 to 0.15 seconds. The number of normal blinking of children is to 5 to 18 times per minute. The numbers of normal blinking of adult male and female are respectively about 20 and 15 times per minute (thus, the lengths of time the children, the adult male, and the adult female remains in a state of opening his or her eyes are respectively 12 to 3.3 seconds, about 3 seconds, and about 4 seconds). In the present invention, a preferably used image involves the blinking speed that is 0.2 to 0.5 times as fast as the normal speed, and the movement of closing the eye carried out with the time of keeping the eyes in a normal opened state being almost 0, and 1 second at the longest (the normal length multiplied by 3 to 12 or longer). Thus, the subject A can more easily recognize a position of the movement.

In the image (iib), the person may move his mouth in a normally speaking manner as shown in Fig. 3D. An image is preferable in which the person intentionally opens and closes his mouth dynamically, so that the large movement of the mouth can be observed. Furthermore, when the subject A is an infant, words luring the reaction such as baby words and hellos are preferable to draw more attention of the subject A to the image. It is a matter of course that the voice can be used in combination with the image.

Thus, the image can be formed that can facilitate an attempt to lead the eye gaze of the subject A to moving portions (eyes, mouth, and their peripheral, in particular).

Next, the predetermined non-human image (II) will be described.

The predetermined non-human image (II) used in the present invention includes as an appearance prediction image (α), an illusion recognition image (β), and a difference search image (γ) each displayed for several seconds, and used together with the predetermined human image (I). At least one of these images is used to form various sequential images. For example, the sequential images may be in a sequence such as (I) → (α) and (β) → (I). When more than one of the images are used, the sequential images may be in a sequence such as (I) → (α) → (β), (γ) → (I) → (β), and (α) → (I) → (β) → (I) → (α).

The appearance prediction image (α) is a moving image involving the following behavior of a picture (referred to as moving body (α1)) that moves on the display portion under a certain rule. Specifically, the moving body is not displayed for a predetermined period of time, when the moving body moves off the screen or when another picture (referred to as hiding body (α2)) displayed on the screen overlaps the moving body, so that it seems as if the moving body is disposed behind the hiding body (α2), and then is redisplayed on the screen. The picture is not particular limited.

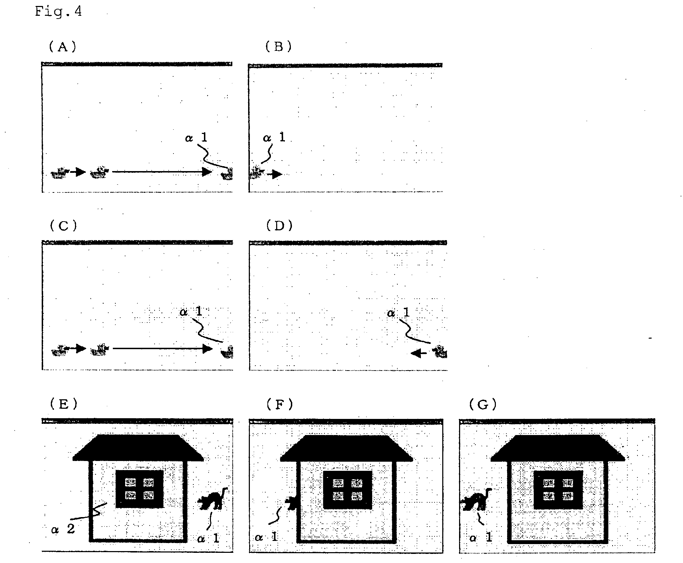

The term "moves under a certain rule" indicates a state where the subject A can easily identify a redisplayed position of the moving body (α1) in the following images. Specifically, an image in Figs. 4A and 4B shows a state of the moving body (α1) on the screen going through a series of operation including appearing on the display portion and moving in a single direction to disappear off the screen, and then being redisplayed at the same position. Image in Figs. 4C and 4D shows a state of the moving body (α1) redisplayed at the position in the opposite direction, that is, the position where the moving body (α1) has disappeared off the screen. In Figs. 4A to 4D, the images with a plurality of arrows and pictures are hypothetically displayed are shown for the sake of description. Actually, a single picture is used, but this does not mean that a plurality of moving bodies cannot be displayed.

Alternatively, Figs. 4E to 4G show a state where the hiding body (α2) is displayed on the screen, and the redisplayed position of the moving body (α1) can be easily identified by the subject A with an image under the following rule. The moving body (α1), in the course of moving on the screen, is overlapped by the hiding body (α2), to be not displayed. Then, the moving body (α1) is redisplayed by passing through the hiding body (α2). The states may be combined.

Thus, the images are configured to enable the eye gaze of the subject A to be intentionally led to a predetermined position (predicted position on the screen) on the image, where the redisplaying occurs.

The illusion recognition image (β) is an image with pictures including an illusion causing element (β1) and a non-illusion causing element ((β2) on the screen of the display portion. Such pictures include illusive images in general sense which are also known as illusive images and illusionary figures [which are described in various forms in the item "optic illusion" in "Wikipedia, The Free Encyclopedia", http://en.wikipedia.org/wiki/Peripheral drift illusion] .

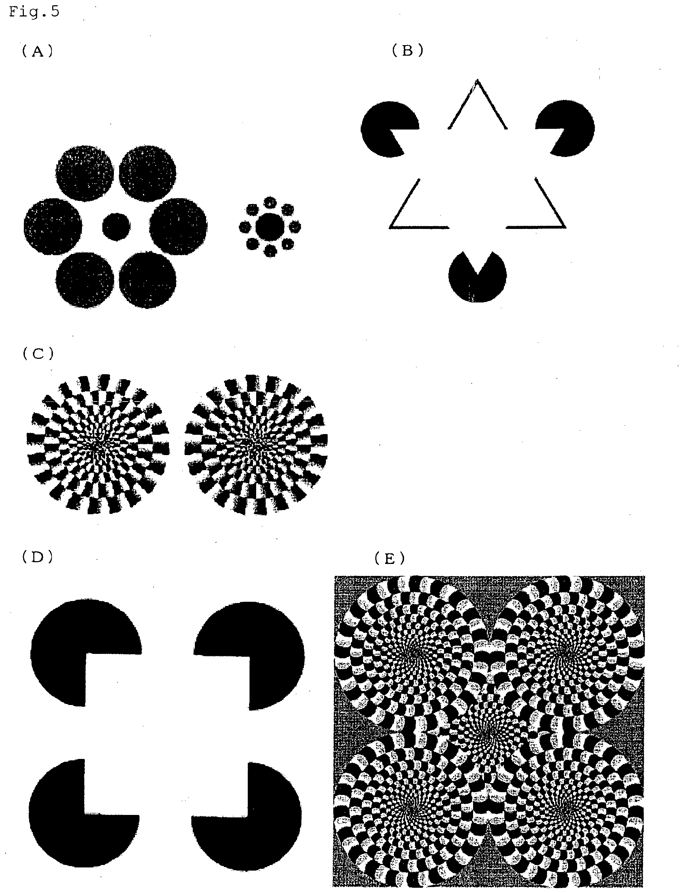

The illusion recognition image (β) includes those shown in Fig. 5. Fig. 5A is an example where an Ebbinghaus illusionary figure is used. Fig. 5B is an example where a Kanizsa triangular illusionary figure is used. Fig. 5C is an example where a Fraser-Wilcox illusionary figure is used.

Furthermore, illusionary figures derived and/or developed from the figured described above may also be used as the picture. Fig. 5D is an example of a developed version of the Kanizsa triangular illusionary figure in Fig. 5B. Fig. 5E is an example of a developed version of the Fraser-Wilcox illusionary figure in Fig. 5C.

When these pictures are used as the illusion recognition image (β) of the present invention, the illusion causing element (β1) and the non-illusion causing element (β2) are displayed as the pictures on the screen of the display portion 21 at the same time.

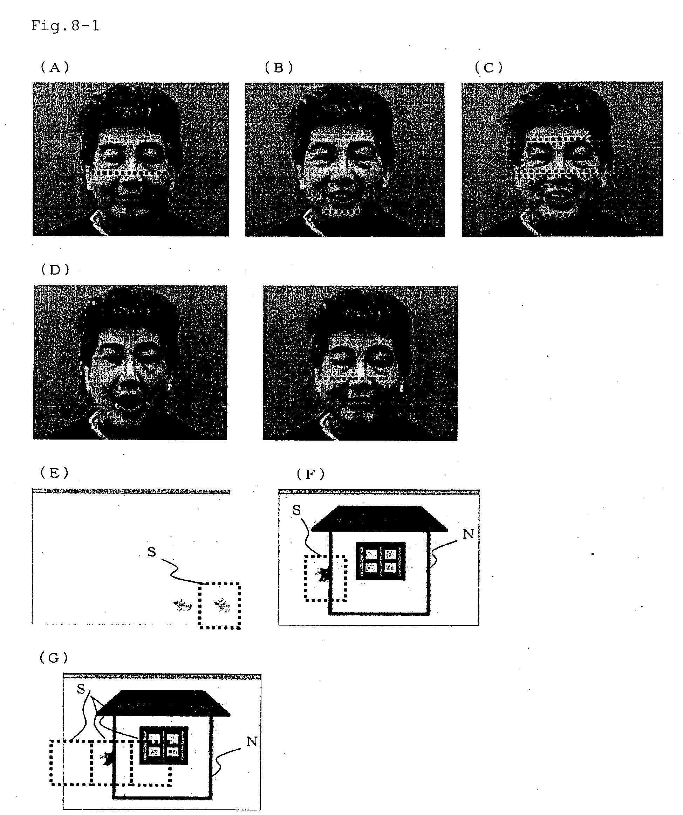

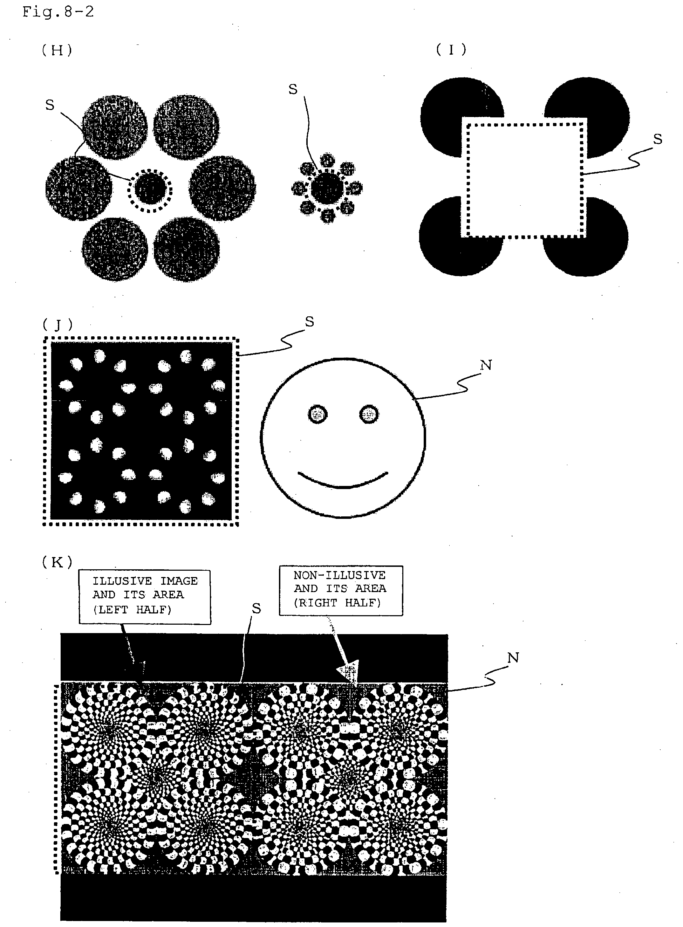

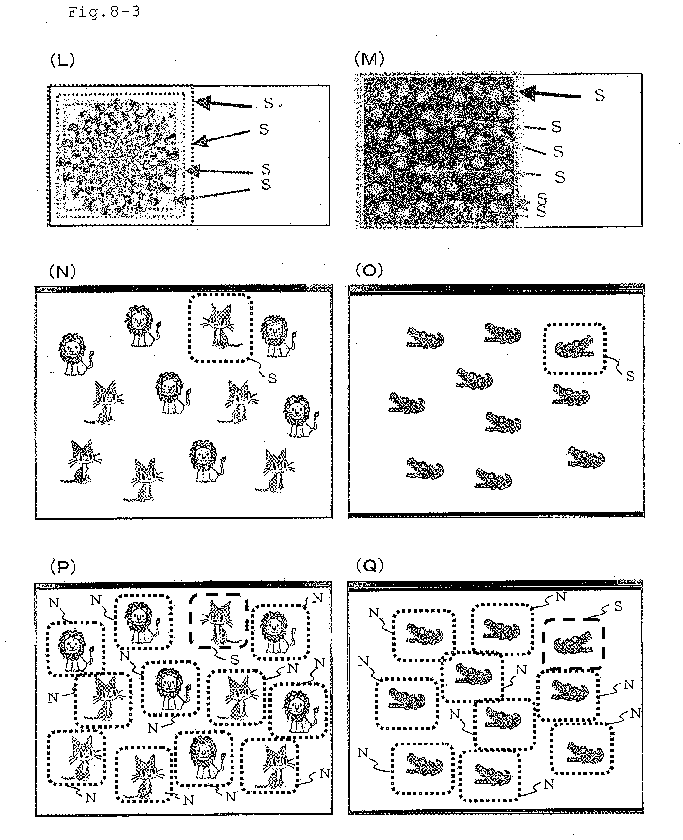

Here, a case where the Ebbinghaus illusionary figure as shown in Fig. 5A is used is shown in Fig. 8H. Specifically, the illusion causing elements (β1) herein are circles respectively at the center of the two sets of circles and causes optical illusion that the center circles seems to be different in size. The non-illusion causing element (β2), as the portion around the illusion causing element (β1), includes circles arranged around the center circle.

A case where the Kanizsa triangular illusionary figures shown in Figs. 5B and 5D are used is shown in Fig. 8I. Specifically, the illusion causing element (β1) is a portion where a shape that does not exist in the screen is displayed, and the non-illusion causing element (β2) is a portion of the picture displayed on the screen.

In the present disclosure, the term "shape that does not exist" does not necessarily represent something that does not exist in the screen or a transparent object. The term represents a space having the same color as the background color of the screen as a whole, and thus cannot be visually distinguished from the background color on the screen.

A case where the Fraser-Wilcox illusionary figures shown in Figs. 5C and 5E are used is shown in Fig. 8K. Specifically, here, the picture as a whole serves as the illusion causing element (β1), and thus the illusion causing element (β1) is set as a portion of the screen (left half of the screen in the figure) where the picture is disposed. The non-illusion causing element (β2) is set as the other portion in the screen (right half of the screen in the figure) where the picture is not displayed.

It seems that the mechanism of these particular illusionary figures has not been completely defined yet. Still, each of these particular illusionary figures is, in common, constructed as a still image and includes an illusion causing element which causes optical illusion that, when the still image is looked at, "a portion of the figure from which the eyes are pulled slightly away seems to be wavering vertically, horizontally, or rotationally" at a portion of the figure around the point which is looked at (i.e., a portion of the figure within the field of view, except for the center of the field of view (therefore, this portion is not visually in focus)) although the image is constructed as a still image.

The effect of this illusion is regarded as substantially the same to any individual looking at this kind of image, unless the individual is visually impaired (i.e., the individual is a color-blind individual, individual with imperfect color vision, sightless individual or the like). For a color-blind individual or individual with imperfect color vision, a grayscale image or image with color(s) which can be recognized by the individual may be selected.

In examples shown in Figs. 8J and 8K, the illusionary figure and the non-illusion causing figure are respectively disposed on left and right sides of the entire display portion.

The non-optical illusion causing figure is a figure which is similar to the illusive image in appearance (i.e., elements of morphology, such as shape, pattern, color and the like) but causes no optic illusion that, when the image is looked at, a portion of the image from which the eyes are pulled slightly away seems to be vertically, horizontally, or rotationally wavering. For example, the illusion causing element can be deleted by changing the pattern of shading (color combination of each dot) so that the positions of shadows therein correspond to the shadows produced by a light source from only one direction.

Thus, for the subject A, the displayed object seemingly includes the identical pictures wholly covering the screen, but the object can be distinguished from each other as the portion causing the optical illusion (left half of the screen in the figure) and the portion causing no optical illusion (right half of the screen in the figure) when the subject A keeps looking at the pictures as a whole.

Specifically, the subject A looks at some portion of the screen displayed on the display portion 21. When an illusive image is displayed, a subject feels optic illusion. The subject A looking at the illusive image feels optic illusion in a predetermined portion of the image, which is a portion of the image from which the eyes are pulled slightly away, when the eye gaze of the subject A is moved. When the subject A feels the optic illusion, the eye gaze is led to the position in which the subject feels optic illusion. However, in the predetermined illusion causing element image, it becomes impossible to feel the optic illusion when the subject A looks at the portion. Thus, the eye gaze of the subject A can be frequently moved on the screen.

However, when the subject is an individual with autism, the subject does not feel optic illusion or looks at, with a strong will, some portion of the image which the subject he or she is interested in. For this reason, a subject with autism naturally tends not to be influenced by the illusion. In other words, the eye gaze of the individuals with autism is less likely to move frequently on the screen.

For easy leading of intended moving of the eye gaze of the subject A to a predetermined position, at least one of the displayed illusive image and non-illusive image may occasionally move horizontally or blink. However, employment of a still image with similar pictures all over the display portion 21 enables detection of more naturally occurring eye gaze movement of the subject A without intended leading of the eye gaze movement of the subject A. In addition, this is especially preferred in the case of an infant where verbal communication is not an option.

Based on the elements described above, other pictures causing the optical illusion may be applied to the image with the configuration where the illusion causing element (β1) and the non-illusion causing element (β2) are displayed on the screen of the display portion at the same time.

Thus, an image is formed that enables leading of the eye gaze movement of the subject A to a predetermined position where the illusion causing element (β1) is displayed.

The difference search image (γ) is an image as a combination of a plurality of identical pictures (γ1) with the same or similar appearance and one or several different pictures (γ2) with the shape different from the identical picture. In the image, the identical pictures (γ1) and the different picture (γ2) are displayed in the display portion in a mixed manner.

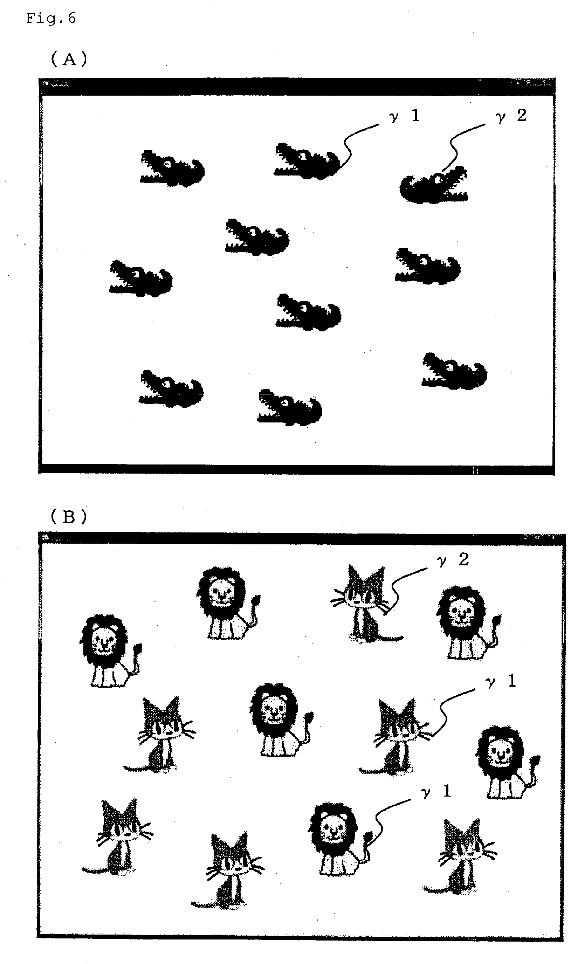

As shown in Fig. 6A, in the image, a plurality of pictures with the same appearance (the identical pictures: γ1) are dispersed on the screen and one or several pictures (different picture γ2) having one of the color or the orientation changed from the identical picture are included. Specifically, in the figure, of 10 alligators, one alligator faces an opposite direction.

Alternatively, as shown in Fig. 6B, a plurality of pictures with the same appearance (the identical pictures: γ1) are dispersed on the screen and one or a several pictures each two or more elements differentiated in respect of the appearance, the orientation, and the color different from those in the identical picture are included. Specifically, in the figure, of 6 lions and 6 cats, only one cat faces an opposite direction.

As described above, a plurality of identical pictures (γ1) and one or several different pictures (γ2) are displayed. As in the illusive image, naturally occurring eye gaze movement of the subject A with a will of the subject himself/herself can be led with no lead to or restriction of the position to be looked at. Specifically, multiple identical pictures seem to be dispersed in the entire screen, and the subject A, with his or her own will, makes the eye-gaze movement to find the different picture in the group of images that seems to be the same, without leading of the eye gaze of the subject A with a stimulation due to the difference in pictures.

Thus, an image is formed that enables the intentional leading of the eye gaze of the subject A to a predetermined position where the identical picture (γ1) or the different picture (γ2) is displayed.

Next, a leading image (θ) displayed together with the combination images will be described.

The leading image (θ) is used to display a picture for leading the eye gaze of the subject A to a predetermined position in advance, before the combination image is displayed on the screen of the display portion 21. The picture is displayed at a predetermined position in the screen for few seconds.

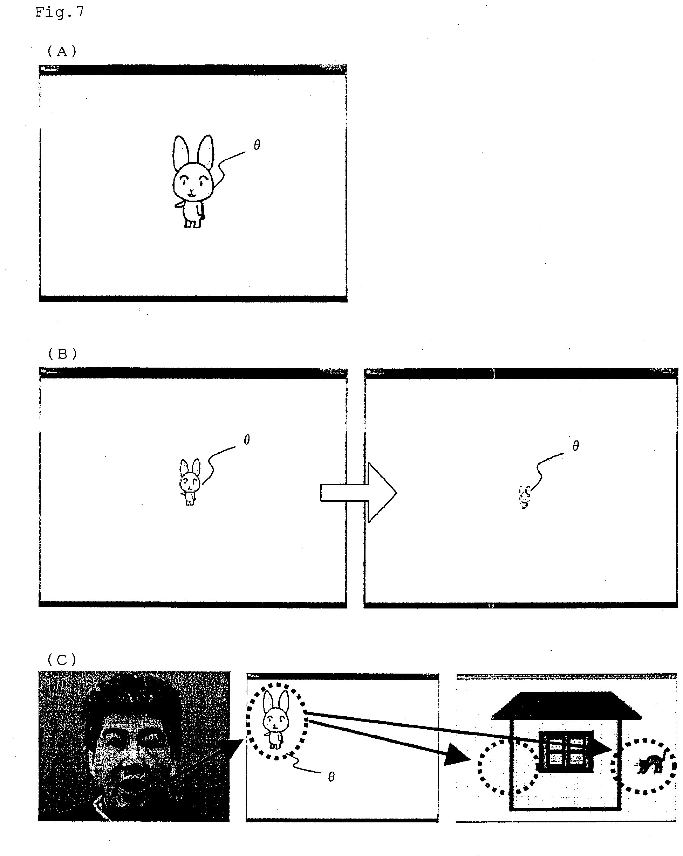

In the leading image (θ), as shown in Fig. 7A, a picture, drawing the attention of the subject A, is displayed in a portion of the screen. Thus, the initial eye-gaze position in the screen to be displayed next is intentionally led to a predetermined position.

The leading image (θ), which is an image used to lead the eye gaze to the predetermined position as described above, may be a still image or a moving image. Preferably, the picture does not have a size too big to be displayed over the entire screen, but has a modestly small size.

As shown in Fig. 7B, the picture enlarged over the entire screen may be gradually downsized to be displayed. Alternatively, the picture may not remain still. Specifically, it is a matter of course that the moving picture may disappear by shrinking at a predetermined position of the screen, or by moving off the screen, since the object thereof is to lead the eye gaze.

What is important herein is that, as shown in Fig. 7C, the leading image (θ) is displayed in relationship with the position for evaluating the eye gaze position in the combination image to be displayed next.

This will be described more in detail. Specifically, to evaluate whether the eye gaze moves to the position of the mouth in the face image (iib) as the predetermined human image (I) where the mouth is opening and closing, the leading image (θ) is displayed at a position away from the mouth so as not to affect the evaluation. Thus, a case where the mouth is coincidentally displayed at a position where the subject A is looking at, that is, the eye gaze position not intended by the subject A at the displayed timing can be excluded from the evaluation. All things considered, the accuracy of the evaluation can be further improved.

Alternatively, to evaluate whether the eye gaze moves to the position of the eyes in the face image (iia) as the predetermined human image (I) where the eyes are opening and closing, the leading image (θ) is intentionally displayed at the position of the eyes. This contributes to an evaluation of the tendency of the individuals with autism to intentionally break eye contact at a moment when the face image is displayed.

The matters described above similarly applies to (α), (β), and (γ) as the predetermined non-human image (II). In the predetermined non-human image (II), the leading image (θ) can also be used as a leading element for insinuating an eye gaze position as the correct position to see. Specifically, the leading image (θ) may be displayed at the same position as the appearance prediction position, the illusion causing position, and the position of the different picture, with the eye gaze position for first several seconds after the displaying prevented from being evaluated.

The autism diagnosis support system uses the eye-gaze detecting unit 2 and the various combination images described above to evaluate the tendency of eye-gaze movement of the subject A as follows.

First, as input means (b) of the autism diagnosis support system, the body unit 1 transmits the displayed image information 31, related to the combination image, to the display portion 21 of the eye-gaze detecting unit 2. Then, the body unit 1 receives the eye-gaze position information 32 of the subject A on the screen, from the eye-gaze detecting unit 2.

The displayed image information 31 includes data on the picture and data on the displayed timing of the combination image. If the combination image has already been prepared on the side of the eye-gaze detecting unit 2, the body unit 1 may transmit an instruction for the displayed timing of the combination image.

The eye-gaze position information 32 is information indicating the eye-gaze position of the subject A described above. The body unit 1 receives the eye-gaze position information 32 as position coordinate data corresponding to the position on the display portion 21 and image data in which an eye-gaze position is indicated by a marker. The eye-gaze position information 32 thus received may be displayed on a display portion 11 of an inspector B to be checked by the inspector B.

Then, as eye-gaze evaluation means (c) of the autism diagnosis support system, the body unit 1 records the eye-gaze position information 32 of the eye-gaze of the subject A corresponding to the combination image, in a storing medium (eye-gaze information storing portion), such as a memory or a hard disk. The eye-gaze position information 32 thus recorded may be displayed on the display portion 11 (eye-gaze position information displaying portion) of the inspector B to be checked by the inspector.

As a result of the processing up to this point, in Fig. 1, the eye-gaze position information 32 and information of result of detection 34 of the eye-gaze position information that are displayed on the screen of the other display portion 11, can be checked by the inspector B in real time or at a later time. A configuration where body unit 1 and the eye-gaze detecting unit 2 are incorporated in the display portion 11 of the subject A or the inspector B may also be employed. As an extreme example of this integrated configuration, under the condition that the diagnosis of the subject A and the checking by the inspector B do not proceed simultaneously, with the operation being switched between that for the subject A and that of the inspector B, all the components (the display portions and the entire body unit 1) may be integrated to be used for both of the operations. There is no limitation on disposing the units on the side of the inspector B and on the side of the subject A in the same chamber. The present disclosure includes inspection of the subject A at a remote location by the inspector B, and evaluation of a recorded inspection image of the subject A by the inspector B at a different place and at a later time.

Then, the eye-gaze evaluation means (c) executes an algorithm for determining whether the subject A is looking at the predetermined position for each image in the combination image, based on the eye-gaze position information 32.

Specifically, the eye-gaze evaluation means (c)of the body unit 1 sets a stimulated area S and a non-stimulated area N described below in the combination image, and obtains the frequency of the eye-gaze movement of the subject A in each of the areas S and N thus set.

The stimulated area S is a range on the screen for determining the tendency that the typically developing individuals in particular is likely to have the eye gaze movement led. The stimulated area S surrounds a moving portion in the predetermined human image (I) and a picture at each predetermined position in the non-predetermined human image (II).

The non-stimulated area N is a range on the screen for determining the tendency that the individual with autism in particular will not have the eye gaze movement led, and is a range that surrounds the picture in a specific position excluding the stimulated area S to which eye-gaze movement is not led.

Furthermore, as will be described later, the stimulated area S and the non-stimulated area N may each be provided in one or a plurality, downsized/enlarged, moved, displayed for only a predetermined period, and exchange positions with each other.

The ranges of the areas S and N are set along the outer shape (contour) of the picture at the predetermined position. As an easy way, the screen of a displaying member is divided in vertical and horizontal directions into grids (blocks) of appropriate sizes. Each of the blocks at a position corresponding to the picture is set as a single subarea, and a single picture may be formed of a set of continuous sub areas. Here, a further determination may be made as appropriate to set a block as the subarea when the block is at least half filled with a part of the contour of the picture, or set a block as the subarea when the block slightly corresponds to the picture. Furthermore, a determination may be made as appropriate to set the entire portion outside the display member 21 as the non-stimulated area N, or as out of area to be excluded from the setting.

The areas S and N may each be set at a predetermined position of the combination image in advance, or at a position corresponding to a predetermined picture in each combination image that is automatically displayed. In the body unit 1, the information of positon of each area in all the combination images is provided for the image as a subject of the displayed image information 31, and stored in the eye-gaze position information. The timing of the provision may be before or after the displayed image is displayed to the subject A, as long as it is before the body unit 1 stores the frequencies of the eye-gaze movement of the subject A as stored information 33, and analyzes the stored information 33.

In the body unit 1, the information may be provided by automatically setting each area information based on color data in the image, a picture determination program, and the like, or by the inspector B manually setting and adjusting each area information for each image.

For each of the areas, the stored information 33 is evaluated with an eye-gaze position evaluation algorithm. The stored information 33 includes, as the frequency of the eye-gaze movement of the subject A, total, average, and maximum eye-gazing times on each area, the number of movements between the areas S and N, and the speed and the direction of the movement, in each displayed period of the combination image.

The eye-gaze position evaluation algorithm for comparing the eye-gaze position information in each combination image with the eye-gaze position information of the individuals with autism and/or the typically developing individuals will be described below.

First, an algorithm for the predetermined human image (I) will be described.

The body unit 1 displays the predetermined human image (I) on the display portion 21 to make the subject A look at the moving portion of the human image. Specifically, from the still image (i) as a state where the face image on the screen remains still as a whole with no moving portion, the face image (iia) where only the eyes are opened and closed and the face image (iib) where only the mouth is opened and closed are displayed. The face image (iia) represents a state where the moving portion is displayed at the position of the eyes. The face image (iib) represents a state where the moving portion is displayed at the position of the mouth. Thus, the tendency of the subject A to look at the position of the moving portion is checked.

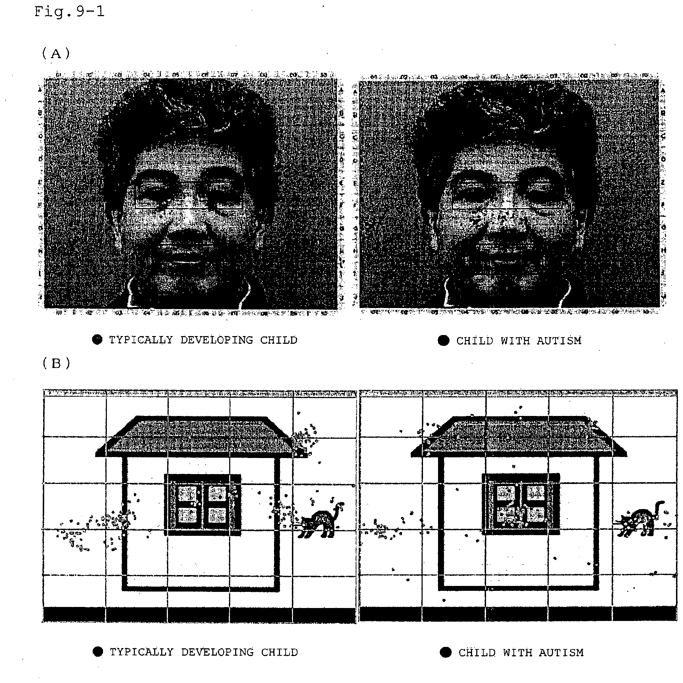

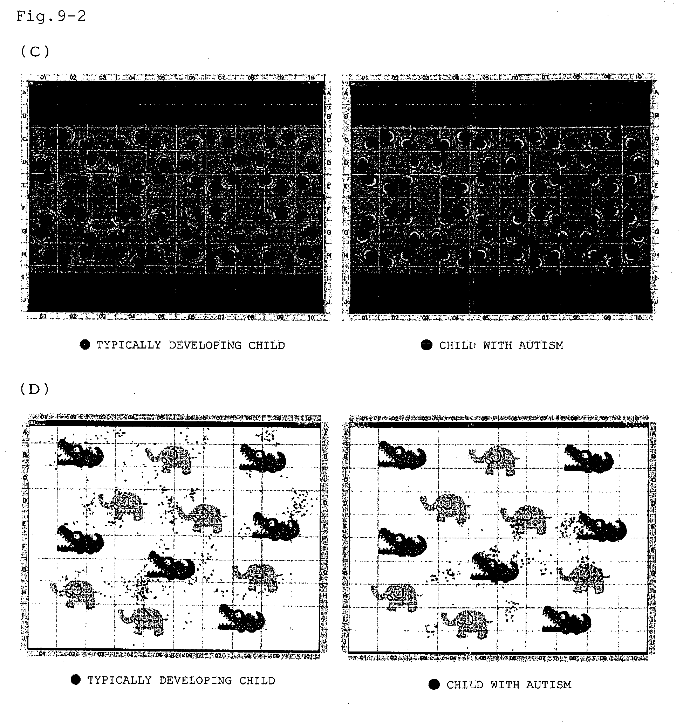

For example, Fig. 9A shows one example in Table 1 described below, where frequencies of the eye-gaze movement of the typically developing individual and the individual with autism are extracted when (iia) is displayed for 5 seconds. The eye-gaze position of the subject is displayed as a plot point on the image once in every 33 milliseconds. The distribution of the dots of the plot point in the figure clearly shows that the individual with autism does not look at the eyes, and his or her eye gaze is concentrated in a portion around the mouth.

Thus, as shown in Fig. 8A, the eye gaze evaluation means (c) sets, in the face image (iia) where only the eyes are opened and closed, the stimulated area S at the position of the eyes, and the non-stimulated area N at the position of the mouth. Thus, the frequency of the eye-gaze position movement of the subject A at the predetermined position in each of the areas S and N is obtained.

Furthermore, as shown in Fig. 8B, the eye gaze evaluation means (c) sets, in the face image (iib) where only the mouth is opened and closed, the stimulated area S at the position of the mouth, and the non-stimulated area N at the position of the eyes. Thus, the frequency of the eye-gaze position movement of the subject A in each of the areas S and N is obtained.

In the still image (i), the positions of both the eyes and the mouth are set as the non-stimulated area N (state with no stimulated area S). As shown in Fig. 8C, the areas may be provided in an overlapping manner to analyze whether the subject is directly looking at the eyes or the mouth. The overlapping areas may be provided to images other than the face image.

**Table 1**

| Displayed state of predetermined human image (face image) | Set area | | Tendency of frequency of eye-gaze movement of typically developed individual (T)/individual with autism (A) |
|---|---|---|---|
| | Around eyes | Around mouth | |
| Still image (i) | Area N | Area N | T: Maximum eye gazing time on the area N is short. |
| | | | The number of movements between the areas N is large. |
| | | | A: Maximum eye gazing time on the area N is long. |
| | | | The number of movements between the areas N is small. |
| Eye moving image (iia) | Area S | Area N | Compared with (i), T: Total and maximum eye gazing times on the area S are long. |
| | | | Total and maximum eye gazing times on the area N are short. |
| | | | The number of movement between the areas S and N is large. |
| | | | Subject looks directly in the eyes in the area S, and increase in the total eye gazing time on a portion around the contour of the eyes tends not to be large. |
| | | | A: Total and maximum eye gazing times on the area S are short. |
| | | | Total and maximum eye gazing times on the area N are long. |
| | | | The number of movement between areas S and N is small. |
| | | | Subject does not look directly in the eyes in the area S, and increase in the total eye gazing time on the portion around the contour of the eyes tends to be large. |
| Mouth moving image (iib) | Area N | Area S | Compared with (iia), T: Total and maximum eye gazing times on the area S are short. |
| | | | Total and maximum eye gazing times on the area N are long. |
| | | | The number of movement between the areas S and N is large. |
| | | | |
| | | | A: Total and maximum eye gazing times on the area S are long. |
| | | | Total and maximum eye gazing times on the area N are short. |
| | | | The number of movement between the areas S and N is small. |

As described above, compared with the typically developing individual, the individual with autism have a certain tendency in any of (ia), (iia), and (iib). Specifically, while the eye-gaze movement to the moving portion, that is, to a portion around the eyes and the mouth as the stimulated area S is found, the individual with autism tend to pay more attention to a certain position he or she is interested in. Thus, obtaining the frequency of the eye-gaze movement not only in the stimulated area S but also in the non-stimulated area N is effective.

In the face image (iib) in particular where only the mouth is opened and closed, both the typically developing individual and the autism tend to bring the eye gaze to a portion around the mouth, as shown in Fig. 8D. Based on this feature, the reversing tendencies of the eye-gaze movement in the stimulated area S and the non-stimulated area N when the face image (iib) where only the mouth is opened and closed is displayed, and then the face image (iia) where only the eyes are opened and closed is displayed, may be analyzed.

Alternatively, the frequency of the movement of the position of the eye while the only the mouth is opened and closed may be extracted in the face image (iib) where only the mouth is opened and closed, because the typically developing individual relatively tends to look at the position of the eye although not elaborated in the figures. Here, the frequency of the movement to the position of the eye while only the mouth is opened and closed may be detected with the portion around the mouth set as the non-stimulated area N, and the portion around the eyes set as the stimulated area S.

With this algorithm, the tendency difference between the typically developing individual and the individual with autism in the frequency of the eye-gaze movement can be extracted. The difference, which contributes to the evaluation, can be extracted based on both common and uncommon elements of the eye-gaze movement of the typically developing individual and the individual with autism.

Next, an algorithm of the predetermined non-human image (II) will be described.

In the appearance prediction image (α), a tendency of the subject A to look at the predetermined portion where the moving body (α1) is redisplayed at a redisplaying timing as shown in Figs. 4A to 4G is checked.

Thus, the eye-gaze evaluation means (c) sets the stimulated area S at each predetermined portion, where the moving body (α1) is redisplayed at the redisplaying timing, as shown in Figs. 8E to 8G. When there is the hiding body (α2), the non-stimulated area N is set at a position surrounding the hiding body (α2). Then, the frequency of the eye-gaze movement of the subject A in each of the areas S and N is obtained. Here, it is important that whether the subject A tends to look at the moving body (α1), and whether the subject A tends to look at the redisplayed portion in a predicting manner after the moving body disappears, are preferably analyzed.

**[Table 2]**

| Displayed state of appearance prediction image | Set area | | | Tendency of frequency of eye-gaze movement of typically developed individual (T)/individual with autism (A) |
|---|---|---|---|---|
| | Position of moving body | Redisplaying position | Position of hiding body | |
| (1) Until displayed moving body disappears (while moving body is moving) | Area S | Area N | Area N | T: Maximum eye gazing time on the area S is long. |
| | | | | The number of movements between the areas S and N is large. |
| | | | | |
| | | | | A: Maximum eye gazing time on the area N is long. |
| | | | | The number of movements between the areas N is small. |
| (2) While moving body is disappeared off screen or behind hiding body | Area N (May not be set when hiding body is provided) | Area S | Area N | Compared with (i), T: Total and maximum eye gazing times on the area S are long. |
| | | | | Total and maximum eye gazing times on the area N are short. |
| | | | | The number of movement between the areas S and N is large. |
| | | | | A: Total and maximum eye gazing times on the area S are short. |
| | | | | Total and maximum eye gazing times on the area N are long. |
| | | | | The number of movement between the areas S and N is small. |
| (3) While moving body is redisplayed (several seconds before/after redisplay timing) | Area N (May not be set when hiding body is provided) | Area S | Area N | Compared with (iia), T: Total and maximum eye gazing times on the area S are long. |
| | | | | Total and maximum eye gazing times on the area N are short. The number of movement between the areas S and N is large. |
| | | | | A: Total and maximum eye gazing times on the area S are short. |
| | | | | Total and maximum eye gazing times on the area N are long. |
| | | | | The number of movement between the areas S and N is small. |
| (4) Until moving body disappears after being redisplayed | Area S | Area N | Area N | Same as (1) described above. |

The stimulated area S set at the predetermined position is set for short period of time before and after the redisplayed timing. The same position is set as the non-stimulated area N or out-of-setting area in other timings . The frequency of the movement may be obtained with the moving body (α1) regarded as the non-stimulated area N or the non-area setting target when the stimulated area S is at the redisplayed position, and as another stimulated area S at other timings.

The position of the stimulated area S at the redisplayed position may be fixed to the redisplayed position, or may be moved in accordance with the movement of the moving body (α1). The period, during which the stimulated area S is set may be adjusted in accordance with the movement speed of the moving body (α1) and reaction delay of the subject A, and may be about 1 or 2 seconds before and after the redisplaying.

As shown in Fig. 8G, a plurality of stimulated areas S adjacent to the stimulated area S may be set. Thus, whether the individual with autism are looking directly at the adjacent stimulated areas S is analyzed to find whether the individual with autism tends to intentionally look positions in front of or behind the moving body rather than looking directly at the moving body, so that he or she does not directly look a person in the eye. The adjacent areas may be provided in cases other than the appearance prediction image.

With this algorithm, the tendency difference between the typically developing individual and the individual with autism in the frequency of the eye-gaze movement can be extracted in the appearance prediction image (α) different from the predetermined human image (I), with the stimulated area S and the non-stimulated area N being changed. The difference contributes to the evaluation achieving high level screening for detecting the individuals with autism without fail.

In the illusive image (β), the tendency to look at the illusion causing element is checked with the illusive images shown in Figs. 5A to 5E displayed by the body unit 1 to make the subject A look at the illusion causing element.

Thus, as shown in Figs. 8H to 8M, the eye-gaze evaluation means (c) sets the stimulated area S at the position of the illusion causing element (β1) and sets the non-stimulated area N at the position of the non-illusion causing element (β2). Then, the frequency of the eye-gaze movement of the subject A at the predetermined position in each of the areas S and N is obtained.

For example, Fig. 9C shows an example in Table 3, where the frequencies of the eye-gaze movement of the typically developing individual and the individuals with autism are obtained with (β1) and (β2) are displayed side by side on left and right sides as shown in Fig. 8K for 5 seconds. The eye-gaze position of the subject is displayed as a plot point on the image once in every 33 milliseconds . The distribution of the dots of the plot point in the figure clearly indicates that the eye gaze of the individual with autism is concentrated at a single point of (β2).

Here, the frequency of the eye-gaze movement can be used to check the tendency and determine whether the subject is looking at the optical illusion with interest, based on the eye-gaze movement in the following manner. Specifically, the total and maximum eye gazing times and the rate of the time in each of the stimulated area S and the non-stimulated area N from the beginning to the end of the displaying are used. It is determined that the subject is not interested in the optical illusion when the number of movements between the areas S and N and the rate of the total time are small.

A case where the Kanizsa triangular illusionary figure as shown in Fig. 8I is used is particularly preferable. Here, in the illusion causing element (β1), when a shape that does not.exist in the screen is recognized, a tendency to intentionally look at a portion around the contour of the shape that does not exist in the screen is caused. As a result, the frequency of the movement to the position of the stimulated area S, that is, the position around the contour of the stimulated area S in particular can be increased, in a case of the typically developing individual.

Although not elaborated in the figure, the frequency of the eye-gaze movement to the position around the contour is checked in detail as follows. Specifically, the stimulated area S shown in Fig. 8I, surrounding the shape that does not exist in the screen, is formed as overlapping areas including two large and small areas S along the contour of the shape that does not exist in the screen. Thus, only the eye-gaze movement between the large and the small areas may be extracted.

A case where the Fraser-Wilcox illusionary figure as shown in Fig. 8K is used is particularly preferable. Here, the figure causes the subject to see the illusion causing element (β1) based on the fact that subject feels as if the illusion causing element (β1) is moving in his or her field of view when looking at the non-illusion causing element (β2) rather than directly looking at the illusion causing element (β1). As a result, the frequency of the movement between the positions of the stimulated area S and the non-stimulated area N can be increased, in a case of the typically developing individual. As shown in Figs. 8L and 8M, each area may be provided in the overlapping structure to analyze the tendency to not look the picture directly, or the area may be divided to analyze the sectional eye-gaze movement tendency in a single picture .

**[Table 3]**

| Displayed state of illusive image | Set area | | Tendency of frequency of eye-gaze movement of typically developed individual (T)/individual with autism (A) |
|---|---|---|---|
| | Position causing optic illusion | Position not causing optic illusion | |
| Fig. 8H Ebbinghaus illusionary figure | Area S | Area N | Common tendency T: Total and maximum eye gazing times on the area S are long. |
| | | | Total and maximum eye gazing times on the area N are short. |
| | | | The number of movement between the areas S and N is large. |
| | | | |
| | | | A: Total and maximum eye gazing times on the area S are short. Total and maximum eye gazing times on the area N are long. |
| | | | The number of movement between the areas S and N is small. |
| Fig. 5I Kanizsa triangular illusionary figure | | | Further tendency T: Total eye gazing time on a portion around a contour of portion where a shape that does not exist in the screen is displayed (shape) tends to be long. |
| | | | |
| | | | T: Total eye gazing time on a portion around a contour of portion where a shape that does not exist in the screen is displayed (shape) tends not to be long. |
| Figs. 8J, 8K Fraser-Wilcox illusionary figure | | | Further tendency T: The number of movement between the areas S and N tends to be large also in the illusive image. |
| | | | |
| | | | T: The number of movement between the areas S and N tends not to be large also in other illusive image. |

The illusive image displayed on the screen of the display portion 21 causes the subject A to look at the illusion causing portion. Here, generally, the eye-gaze movement unique to the individual with autism can be checked from the tendency of the subject A in Table 1.

Thus, an image is formed in which the eye gaze of the subject A can be intentionally led to the predetermined position causing the optical illusion in the screen, due to the feeling of strangeness brought by the illusion causing element and the non-illusion causing element.

In the difference search image (γ), a plurality of identical pictures (γ1) and one or several different picture (γ2) as shown in Figs. 6A and 6B are displayed by the body unit 1, and the tendency of the subject A to look at the positions of the identical picture (γ1) and the different picture (γ2) is checked.

Thus, the eye-gaze evaluation means (c) may set the stimulated area S at the position of the different picture (γ2) as shown in Figs. 8N to 8O. The eye-gaze evaluation means (c) further sets the non-stimulated area N at the position of the identical picture (γ1), as shown in Figs. 8P to 8Q. Thus, the frequency of the eye-gaze movement of the subject A in each of the areas S and N is obtained. The non-stimulated area N may not be set at the position of the identical picture (γ1). Here, the non-stimulated area N may be set as any position other than the stimulated area S at the position of the different picture (γ2).

For example, Fig. 9A shows one example in Table 4 described below, where the frequencies of the eye-gaze movement of the typically developing individuals and the individuals with autism are extracted in a case where the Fig. 8Q is displayed for 5 seconds . The eye-gaze position of the subject is displayed as a plot point on the image once in every 33 milliseconds. The distribution of the dots of the plot point in the figure clearly shows that the individuals with autism tend not to look at the pictures in the entire screen.

Here, the frequency of the eye-gaze movement can be checked in the following manner from the eye-gaze movement. Specifically, whether the subject has found the different picture (γ2) can be determined from the total and maximum eye gazing times on the stimulated area S during a period between the start and the end of the displaying. It can be determined that the subject has not yet found the different picture (γ2) and thus is in the processing of searching when the momently eye-gaze movement in the stimulated area S is found. Whether the subject tends to try to find a small number of different picture (γ2) in various pictures or tends to focus on a single picture can be determined as follows. Specifically, the tendencies are checked based on the number of times of the movement and the ratio of the total time between the non-stimulated areas N set to the identical pictures (β1) or between the area S and the area N.

**[Table 4]**

| Displayed state of difference search image | Set area | | Tendency of frequency of eye-gaze movement of typically developed individual (T)/individual with autism (A) |
|---|---|---|---|
| | Position of different picture | Position of identical picture | |
| Figs. 8N and 8O Different picture is set as area S and other portion is set as area N | Area S | Area N | Common tendency *T*: Time it takes to find the area S is long (normal). |
| | | | In addition, the number of movement between the areas S and N is large (move many times between pictures). |
| | | | |
| | | | A: Time it takes to find the area S is notably shorter than the average time. |
| | | | Alternatively, the total eye gazing time on the area S is notably short (if not 0). |
| | | | In addition, the number of movement between the areas S and N is small. |
| Figs. 8P and 8Q Different picture is set as area S and identical picture is set as area N | | | Further tendency: |
| | | | T: The eye gazing time on a single area N or an area other than the areas S and N is short. |
| | | | The number of movement between the areas N is large. |
| | | | He or she looks directly at the picture in the areas S and N and thus the total eye gazing time on a portion around the contour of the picture tends not to be long. |
| | | | |
| | | | A: The eye gazing time on a single area N or an area other than the areas S and N is long. |
| | | | The number of movement between the areas N is small. |
| | | | He or she does not look directly at the picture in the areas S and N and thus the total eye gazing time on a portion around the contour of the picture tends to be long. |

All things considered, the subject looking at images displayed in the entire screen of the display portion with his or her will looks at the different picture found in the identical pictures that seem to be the same (because the subject is interested in the feeling of strangeness/unnaturalness brought by the a portion where a picture seems to be the same but is different in appearance) . This can cause the eye-gaze movement of the subject A between the identical picture and the different picture. It is effective to obtain the frequency of the eye-gaze movement by thus checking the tendencies of the eye-gaze movement of the individual with autism and the typically developing individual.

With this algorithm, the tendency difference between the typically developing individual and the individual with autism in the frequency of the eye-gaze movement can be extracted. The difference, which contributes to the evaluation, can be extracted based on both common and uncommon elements of the eye-gaze movement of the typically developing individual and the individual with autism.

How much the subject A is paying attention to the predetermined area can be recognized through the calculation for each area in the images described above.

The stimulated area S and the non-stimulated area N might be provided in a plurality in a single image, or a plurality of images each including the stimulated area S and the non-stimulated area N might be used. In such cases, the total number of each of the areas S and N may be calculated. Alternatively, the calculation may be independently carried out for, for example, each of the areas such as a stimulated area S1 and a stimulated area S2. Specifically, such a calculation is preferable because according to the average number of staying the eye gaze might move from one area to the other and then returns to and be concentrated in the original area.

Alternatively, the distance of eye gaze movement between two dots in the position information may be converted into the direction and magnitude of a vector to calculate, as a single piece of information, the frequency and/or speed of the eye gaze movement.

As apparent from the above, setting of these areas at the predetermined positions in the image and extracting information of eye-gaze movement at the positions enables easy calculation of tendency of movement based on the stimulated area S such as the tendency of retention of eye gaze within the stimulated area S and the tendency of eye gaze movement into the stimulated area S from an area outside the stimulated area S, or from the stimulated area S into an area outside the stimulated area S based on the eye-gaze position information on the subject A, which calculation may contribute effective analysis and/or comparison.

As will be described later, the stored information in the body unit 1 enables detection of change in the tendency of eye gaze movement relative to previously detected tendency of the same subj ect A using the same image information, or the tendency difference in the eye gaze movement between different subjects A using the same image information.

Comparison of the stored information with previously obtained stored information of an individual who has already definitely diagnosed as a typically developing individual/individual with autism using the same image information also becomes easy.

As described above, the eye-gaze evaluation means (c) stores the result of executing the algorithm on each image in the combination image as the stored information, and performs calculation to determine, in real time or after the completion of detection, whether the detected eye-gaze movement is specific to an individual with autism or a typically developing individual, from the entire combination image. After the calculation, the eye-gaze evaluation means (c) stores the result of the evaluation in the evaluation result storing portion. Optionally, the subject A performs comparison of the detected eye-gaze movement with standard eye-gaze movement of an individual identified as a typically developing individual (or individual with autism), based on the difference from the frequency of the eye-gaze movement unique to the individuals with autism (eye-gaze position information evaluating portion).

Stored information is information obtained as follows. Specifically, the body unit 1 stores the displayed image information (including the area information) which is transmitted to the display portion 21 and eye-gaze position information on the subject A input from the eye-gaze detecting unit 2, and removes inconsistency between the position information pieces to be then stored as the data to be analyzed which is related to eye-gaze positions.

Such stored information 33 may provide improved convenience in that: when a subject A is definitely diagnosed as a typically developing individual/individual with autism later, the diagnosis may be stored in the previous stored information as supplemental information to reinforce the effectiveness of the stored information as reference/compared information, and an examination itself for obtaining stored information can be conducted, even in an area with a small number of experts (specialists) or in a case with no expert (specialist) present such as a group examination in a school or local health center. The definite diagnosis can be made by an expert (specialist), later or immediately for early detection, based on the evaluation result of the stored information, even in a distant place using communicating means. Furthermore, a doctor of another medical field or the like can make the recommendation to obtain definite diagnosis by an expert (specialist) based on the evaluation result of the stored information, accompanied by notice of the stored information to the expert (specialist).

In such information pieces, for further investigation which the inspector desires, it is preferred to add various types of supplemental information with which the reference/target for comparison can be classified various elements, indicating tendency, such as age, gender, or features in appearance.

It is preferred to set a threshold value for the frequency of mutual eye-gaze movement between an area of the plane of an illusion-causing image in which the illusive image is displayed and an area of the plane of an illusion-causing image in which the illusive image is not displayed, based on a database having stored therein previously obtained eye-gaze position information of subjects A and definite diagnosis of each of the subjects A as to whether or not the subject A is an individual with autism.

Any supplemental information other than the above-mentioned position-in-area information and the eye-gaze position information may be added to the stored information. Examples of such supplemental information include the title and property information of the image file, history of display time of the image, information of the specification of the unit used, personal information on the subject A, history of previous diagnoses and the like.

The analysis may be conducted totally for all images displayed to the subject or separately for each of a specific unit of period of time of examination or for each image displayed.

If necessary, it is preferred to conduct calculation for obtaining the difference between the results and the stored information previously obtained for the typically developing individual and/or the individual with autism to be a reference/target for using the same images.

In this analysis, the following aspects are important. Specifically, the predetermined areas are set in a predetermined image. Such setting gives a criterion for calculation of the frequency of eye-gaze movement of the subject A. Furthermore, stored information of the typically developing individual and/or the individual with autism is used for the contrast or difference in tendency of eye-gaze movement. Thus, the frequency of the eye-gaze movement of the subject A can be evaluated. Thus, in such an evaluation, for comparing the frequencies of the eye-gaze movement, the body unit 1 preferably stores various pieces of the eye-gaze position information of the same inspector that has performed the inspection in the past and the eye-gaze position information in the same image of the other subject A. The comparison with the eye-gaze information of a person diagnosed to have autism (definite diagnosis information) as the diagnosis information is particularly preferable.

Preferably, such information can be accumulated, added, and corrected in the stored information. The eye-gaze position information on the subject A may be independently compared with the eye-gaze position information by thus stored in the body unit 1 or may be compared with the average value of the stored specific information, as appropriate.

The difference among the images in the displayed speed and total displayed time leads to the error in the comparison between the total times. Thus, the frequencies in the stimulated area S and the non-stimulated area N per average time may be identified. Furthermore, information related to the display member may be stored in the eye-gaze position information storing portion to prevent the dimensional difference, due to difference of the used display member in the size, the rate, and the resolution, from causing the error in the comparison.

Here, it is preferred that time information, such as information of actual time determined by an atomic clock or the like, which clearly shows the time when the subject A looks at a certain position of the displayed image information to the body unit 1 or the eye-gaze detecting unit 2 is added to the detected eye-gaze position information, since such information ensures consistency of the stored information later. The unit of evaluation is not limited to time and may be determined as appropriate. For example, the eye-gaze detecting unit 2 may perform the calculation for a number of times corresponding the number of detections per second. For example, a single measurement time period necessary for the eye-gaze detecting unit 2 to obtain a single eye-gaze coordinate may be counted as the time of a single plot (for example, with respect to the eye-gaze detecting unit detecting 30 plots per second, 1/30 sec per plot).

The eye-gaze position information evaluation unit may perform the evaluation with all the combination images in the total number of displayed images, or perform the evaluation with the displaying stopped as soon as a predetermined level of frequency of the eye-gaze movement unique to autism is obtained.

Then, the result of the analysis is output to the display portion A as the detection result information (evaluation result outputting portion).

The detected result information is information of the content of analysis of the stored information to be displayed to the inspector, printed or transferred in a format which the inspector desires, in the body unit 1, and instead of being provided only to the display portion, the detected result information may also be output to a printing medium such as a printer, or a storage medium, a reproduction medium, or another display medium provided outside, in a form of screen information or recorded information.

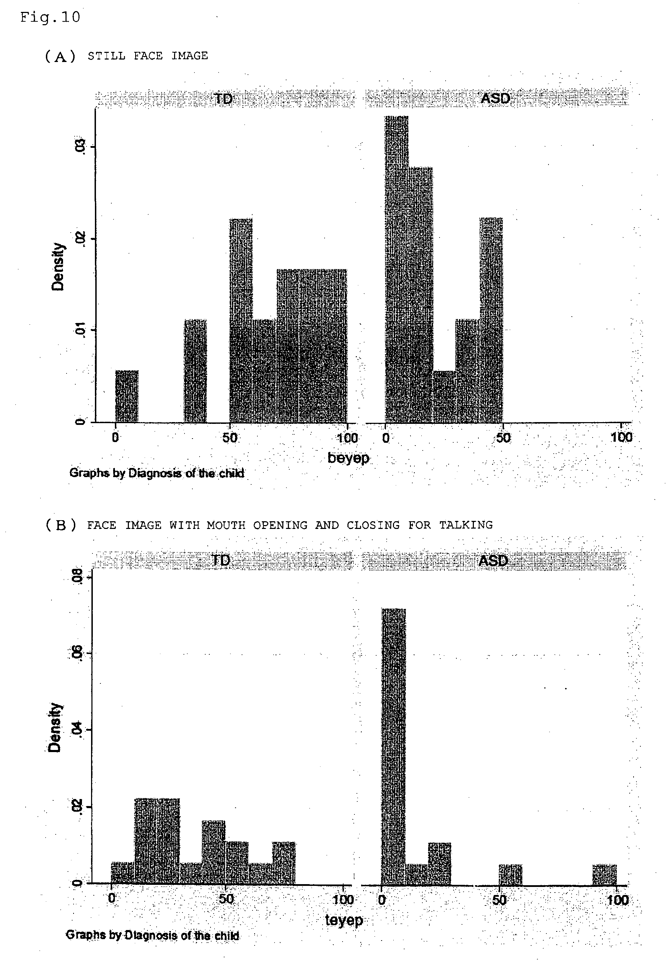

The detection result information may not necessarily be in a form of numeral data, and may be displayed in the form of various kinds of figures or graphs, such as a line graph, bubble chart, scatter diagram, or circle graph, instead of bar diagram as shown in Figs. 10A and 10B. The bar graph represents density distribution obtained by the number and the time of the concentrated eye gaze. This information may be expressed in other way than the content of analysis of the stored information. For example, the information may be expressed as shown in Figs. 9A to 9D. Specifically, the image may be expressed as a moving image recorded as the displayed image information and the eye-gaze position image or with eye gaze movement reproduced in various manners, such as superimposed trajectory of eye gaze movement with changed color.

The graphs shown in Figs. 10A and 10B show the positions of the eye gaze on the stimulated area S and the non-stimulated area N while the combination image is displayed, in the ratio per time of the stay in the area. Here, the calculation is performed on 10 typically developing individuals and 10 individuals with autism, with a case where the subject is constantly looking at the stimulated area S defined as 100%.

Fig. 10A shows tendencies of the eye-gaze movement in the still face image as the still image (i). Fig. 10B shows a state where the tendency of the frequency of the eye-gaze movement to a periphery of the eyes have largely changed while the moving image (iia) of the face where only the mouth is opened and closed, compared with that in Fig. 10A. Here, it can be found that the change in the tendencies of the eye-gaze movement of the typically developing individuals have notably changed from (i) to (iia).

Then, whether the tendency of the frequency of the eye-gaze movement of the inspector is close to the tendency of the frequency of the eye-gaze movement of the typically developing individuals or the individuals with autism is checked. Thus, how much the subj ect is likely to have autism can be checked. The inspector is visually provided with the contrast or difference in tendency of eye-gaze movement between the typically developing individual and the individual with autism, for each image in the combination image or the entire combination image. Thus, supporting to facilitating comparison and investigation can be achieved.

It is preferred that the data presented for comparison is appropriately selected as one of data on each measured individual, an average value and a standard value calculated from data selected under a predetermined condition, or a combination of these.

The above-mentioned manner of presentation of the content of analysis of the stored information is suitable as the eye-gaze position information to show potential symptom (s) of autism to the inspector.

### INDUSTRIAL APPLICABILITY

The method and system of the present invention for supporting autism diagnosis is advantageous in that support can be provided for early detection and early definite diagnosis of autism, even in an infant before the age that the identification by a specialist as an individual with autism can be applied to, based on objective evaluation.

### EXPLANATION OF REFERENCE NUMERALS

- 1: body unit
- 11: display portion
- 2: eye-gaze detecting unit
- 21: display portion
- 22: camera portion
- 23: supplemental imaging lighting portion
- 31: displayed image information
- 32: eye-gaze position information
- 33: stored information
- 34: detection result information
- A: subject
- B: inspector

## Claims

1. An autism diagnosis support device supporting autism diagnosis,
the device comprising:
(i) an eye-gaze detecting portion adapted to display, on a display portion (a3) to be disposed at a position in an eye-gaze direction of a subject, a combination image for sequentially displaying at least two images including a predetermined human image (I) and a predetermined non-human image (II), and adapted to detect eye-gaze position information on the subject looking at the combination image;
(ii) an eye-gaze position information storing portion adapted to store the eye-gaze position information detected by the eye-gaze detecting portion;
(iii) an eye-gaze position information displaying portion adapted to display the eye-gaze position information on the subject stored in the eye-gaze position information storing portion;
(iv) an eye-gaze position information evaluating portion adapted to evaluate the eye-gaze position information on the subject by comparing the eye-gaze position information on the subject displayed on the eye-gaze position information displaying portion with the eye-gaze position information on the individual with autism and/or the typically developing individual;
(v) an evaluation result outputting portion adapted to output an evaluation result obtained by the eye-gaze position information evaluating portion; and
(vi) an evaluation result storing portion adapted to store the evaluation result output from the evaluation result outputting portion or the evaluation result obtained by the eye-gaze position information evaluating portion,
wherein the predetermined non-human image (II) includes at least one type selected from an appearance prediction image (α), an illusion recognition image (β), and a difference search image (γ),
wherein the appearance prediction image (α) is a moving image including a moving body image (α1) that is a picture moving on the display portion (a3) under a certain rule, wherein the moving image is not displayed for a predetermined period of time, when the moving body moves off the screen or when a hiding body image (α2), which is another picture displayed on the screen, overlaps the moving body image (α1), so that it seems as if the moving body is disposed behind the hiding body image (α2), and then is redisplayed on the screen,
wherein the illusion recognition image (β) is an image formed of pictures including an illusion causing element (β1) and a non-illusion causing element (β2) displayed on the screen of the display portion (a3), and
wherein the difference search image (γ) is an image formed of a combination of a plurality of identical pictures (γ1) having the same or similar appearance, and one or several different pictures (γ2) having a shape different from those of the identical pictures, and the identical pictures (γ1) and the different pictures (γ2) are displyed in a mixed manner on the display portion (a3).

2. The autism diagnosis support device according to claim 1,
wherein the predetermined non-human image (II) includes an appearance prediction image (α), an illusion recognition image (β), and a difference search image (γ).

3. The autism diagnosis support device according to any claim 1 or 2,
wherein the eye-gaze detecting portion is adapted to detect eye-gaze position information on the subject looking at a screen of the display portion (a3), by using a camera portion (a1) capturing an image of an eye of the subject, or an electrode portion (a2) to be mounted on a head of the subject and detect a movement of the eye of the subject.

4. The autism diagnosis support device according to any one of claims 1 to 3,
wherein the evaluation result outputting portion is adapted to display an evaluation result of the eye-gaze position of the subject.

5. The autism diagnosis support device according to any one of claims 1 to 4,
wherein the predetermined human image (I) includes a still image (i) and a moving image (ii) partially moving, and
wherein the eye-gaze position information evaluating portion evaluates a frequency of an eye-gaze movement, in a case where the still image (i) or the moving image (ii) partially moving is displayed on the screen of the display portion (a3), on the basis of a contrast or difference between the typically developing individual and the individual with autism in terms of a tendency of an eye-gaze movement where the frequency of the eye-gaze movement of the typically developing individual to a moving portion of the moving image is high but that of the individual with autism is low.

6. The autism diagnosis support device according to claim 5,
wherein the predetermined human image (I) includes three types of images, which are a still image (ia) of a face, a moving image (iia) of the face where only an eye is opened and closed, and a moving image (iib) of the face where only a mouth is opened and closed.

7. The autism diagnosis support device according to claim 6,
wherein the frequency of the eye-gaze movement is evaluated based on a contrast or difference between the typically developing individual and the individual with autism in terms of a tendency of an eye-gaze movement where, while the moving image (iia) of the face where only the eye is opened and closed is displayed, the frequency of the eye-gaze movement of the typically developing individual to a periphery of the eye is high but that of the individual with autism is low.

8. The autism diagnosis support device according to claim 6,
wherein the frequency of the eye-gaze movement is evaluated based on a contrast or difference between the typically developing individual and the individual with autism in terms of a tendency of an eye-gaze movement where the frequency of the eye-gaze movement of the individual with autism to (iia) in a case where the moving image (iib) of the face where only the mouth is opened and closed is first displayed and then the moving image (iia) of the face where only the eye is opened and closed is displayed is low compared with that of the typically developing individual in a case where the still image (ia) of the face or the moving image (iib) of the face where only the mouth is opened and closed is displayed.

9. The autism diagnosis support device according to any one of claims 1 to 8,
wherein an image of a person whom the subject knows is used as the predetermined human image (I).

10. The autism diagnosis support device according to claim 1,
wherein the predetermined non-human image (II) includes the appearance prediction image (α), and
wherein the eye-gaze position information evaluating portion evaluates a frequency of an eye-gaze movement at a time of redisplaying of the moving body image (α1) at a predetermined position in the display portion (a3), after first displaying the moving body image (α1) in such a manner as to move on a screen on the display portion (a3) and then making the moving body image (α1) transition to a non-displayed state by being off the screen of the display portion (a3) or by the hiding body image (α2), based on a contrast or difference between the typically developing individual and the individual with autism in terms of a tendency of an eye-gaze movement where the frequency of the eye-gaze movement of the typically developing individual to a position where the moving body image (α1) is redisplayed is high but that of the individual with autism is low.

11. The autism diagnosis support device according to claim 10,
wherein the eye-gaze position information evaluating portion does not use the frequency of the eye-gaze movement at the time of redisplaying for the evaluation of the frequency of the movement when the redisplaying is implemented for a first time, but uses for the evaluation of the frequency of the movement when the redisplaying is implemented for a second time or after, where a movement, under a certain rule, of a moving body image (α1) is predictable.

12. The autism diagnosis support device according to claim 1,
wherein the predetermined non-human image (II) includes the illusion recognition image (β), and
wherein the eye-gaze position information evaluating portion evaluates a frequency of an eye-gaze movement, in a case where the illusion causing element
(β1) is displayed, based on a contrast or difference between the typically developing individual and the individual with autism in terms of a tendency of an eye-gaze movement where the frequency of the eye-gaze movement of the typically developing individual between a position where the illusion causing element (β1) is displayed and a position where the non-illusion causing element (β2) is displayed is high, but that of the individual with autism is low.

13. The autism diagnosis support device according to claim 1,
wherein the predetermined non-human image (II) includes the difference search image (γ), and
wherein the eye-gaze position information evaluating portion evaluates a frequency of an eye-gaze movement, in a case where the identical pictures (γ1) and the different pictures (γ2) are displayed in a mixed manner on the display portion (a3), based on a contrast or difference between the typically developing individual and the individual with autism in terms of a tendency of an eye-gaze movement where the frequency of the eye-gaze movement of the typically developing individual between a position where the identical picture (γ1) is displayed and a position where the different picture (γ2) is displayed is low, but that of the individual with autism is high.

14. The autism diagnosis support device according to claim 1,
wherein before the combination image is displayed on a screen of the display portion (a3), a preliminary image leading image (θ) is displayed on the screen to lead the eye gaze of the subject to a predetermined position in advance.

## Patentansprüche

1. Vorrichtung zur Unterstützung der Diagnose von Autismus, welche die Diagnose von Autismus unterstützt,
wobei die Vorrichtung umfasst:
(i) einen Teilbereich zum Erfassen des Blickes eines Auges, der ausgelegt ist, auf einem Anzeigeabschnitt (a3), der an einer Position in einer Blickrichtung des Auges eines Subjekts anzuordnen ist, ein Kombinationsbild zum sequentiellen Anzeigen von mindestens zwei Bildern, einschließlich eines vorbestimmten menschlichen Bildes (I) und eines vorbestimmten nicht-menschlichen Bildes (II), anzuzeigen, um Informationen über die Position des Blickes des Auges des Subjekts, welches das Kombinationsbild betrachtet, zu erfassen;
(ii) einen Teilbereich zum Speichern der Informationen über die Position des Blickes des Auges, der ausgelegt ist, die Informationen über die Position des Blickes des Auges zu speichern, die von dem Teilbereich zum Erfassen des Blickes des Auges erfasst wird;
(iii) einen Teilbereich zum Anzeigen der Informationen über die Position des Blickes des Auges, der ausgelegt ist, die in dem Teilbereich zum Speichern der Informationen über die Position des Blickes des Auges gespeicherten Informationen über die Position des Blickes des Auges anzuzeigen;
(iv) einen Teilbereich zum Auswerten der Informationen über die Position des Blickes des Auges, der ausgelegt ist, die Informationen über die Position des Blickes des Auges des Subjekts durch Vergleichen der Informationen über die Position des Blickes des Auges des Subjekts, die auf dem Teilbereich zum Anzeigen der Informationen über die Position des Blickes des Auges angezeigt werden, mit den Informationen über die Position des Blickes des Auges eines Individuums mit Autismus und/oder eines typischen sich entwickelnden Individuums, auszuwerten.
(v) einen Teilbereich zum Ausgeben eines Auswertungsergebnisses, der ausgelegt ist, ein Auswertungsergebnis auszugeben, das durch den Teilbereich zum Auswerten der Informationen über die Position des Blickes des Auges erhalten wurde; und
(vi) einen Teilbereich zum Speichern eines Auswertungsergebnisses, der ausgelegt ist, das vom Teilbereich zum Ausgeben eines Auswertungsergebnisses erhaltene Auswertungsergebnis oder das vom Teilbereich zum Auswerten der Informationen über die Position des Blickes des Auges erhaltene Auswertungsergebnis zu speichern;
wobei das vorbestimmte nicht-menschliche Bild (II) mindestens eine Art einschließt, ausgewählt aus einem Erscheinungsvorhersagebild (α), einem Illusionserkennungsbild (β) und einem Differenzsuchbild (γ),
wobei das Erscheinungsvorhersagebild (α) ein bewegtes Bild ist, das ein Bild (α1) eines sich bewegenden Körpers enthält, welches ein Bild ist, das sich auf dem Anzeigeabschnitt (a3) nach einer bestimmten Regel bewegt, wobei das bewegte Bild für eine vorher festgelegte Zeitdauer nicht angezeigt wird, wenn sich der sich bewegende Körper aus dem Bildschirm bewegt oder wenn ein Bild eines sich versteckenden Körpers (a2), das ein anderes auf dem Bildschirm angezeigtes Bild ist, das Bild des sich bewegenden Körpers (α1) überlappt, so dass es scheint, als ob der sich bewegende Körper hinter dem Bild des sich versteckenden Körpers (α2) angeordnet ist und dann erneut auf dem Bildschirm angezeigt wird,
wobei das Illusionserkennungsbild (β) ein Bild ist, das aus Bildern gebildet ist, die ein eine Illusion verursachendes Element (β1) und ein keine Illusion verursachendes Element (β2) enthalten, die auf dem Bildschirm des Anzeigeabschnitts (a3) angezeigt werden, und
wobei das Differenzsuchbild (γ) ein Bild ist, das aus einer Kombination einer Vielzahl von identischen Bildern (γ1), die das gleiche oder ein ähnliches Aussehen haben, und einem oder mehreren unterschiedlichen Bildern (γ2), die eine Form aufweisen, die sich von denen der identischen Bilder unterscheidet, gebildet ist, und die identischen Bilder (γ1) und die unterschiedlichen Bilder (γ2) in einer gemischten Weise auf dem Anzeigeabschnitt (a3) angezeigt werden.

2. Vorrichtung zur Unterstützung der Diagnose von Autismus nach Anspruch 1, wobei das vorbestimmte nicht-menschliche Bild (II) ein Erscheinungsvorhersagebild (α), ein Illusionserkennungsbild (β) und ein Differenzsuchbild (γ)enthält.

3. Vorrichtung zur Unterstützung der Diagnose von Autismus nach einem der Ansprüche 1 oder 2,
wobei der Teilbereich zum Erfassen des Blickes eines Auges ausgelegt ist, Informationen über die Position des Blickes des Auges des Subjekts, welches auf einen Bildschirm des Anzeigeabschnitts (a3) schaut, zu erfassen, indem ein Kameraabschnitt (a1), der ein Bild eines Auges des Subjekts aufnimmt, oder ein Elektrodenabschnitt (a2) verwendet wird, der auf einem Kopf des Subjekts zu befestigen ist, und eine Bewegung des Auges des Subjekts zu erfassen.

4. Vorrichtung zur Unterstützung der Diagnose von Autismus nach einem der Ansprüche 1 bis 3,
wobei der Teilbereich zum Ausgeben eines Auswertungsergebnisses ausgelegt ist, ein Auswertungsergebnis der Position des Blickes des Auges des Subjekts anzuzeigen.

5. Vorrichtung zur Unterstützung der Diagnose von Autismus nach einem der Ansprüche 1 bis 4,
wobei das vorbestimmte menschliche Bild (I) ein Standbild (i) und ein bewegtes Bild (ii), das sich teilweise bewegt, enthält, und
wobei der Teilbereich zum Auswerten der Informationen über die Position des Blickes des Auges eine Häufigkeit einer Bewegung des Blickes des Auges in einem Fall, in dem das Standbild (i) oder das bewegte Bild (ii), das sich teilweise bewegt, auf dem Bildschirm des Anzeigeabschnitts (a3) angezeigt wird, auf der Grundlage eines Kontrasts oder Unterschieds zwischen dem typischen sich entwickelnden Individuum und dem Individuum mit Autismus in Bezug auf eine Tendenz einer Bewegung des Blickes des Auges, auswertet, wobei die Häufigkeit der Bewegung des Blickes des Auges des typischen sich entwickelnden Individuums zu einem sich bewegenden Abschnitt des sich bewegenden Bildes hoch, die des Individuums mit Autismus aber niedrig ist.

6. Vorrichtung zur Unterstützung der Diagnose von Autismus nach Anspruch 5,
wobei das vorbestimmte menschliche Bild (I) drei Arten von Bildern enthält, die ein Standbild (ia) eines Gesichts, ein bewegtes Bild (iia) des Gesichts, bei dem nur ein Auge geöffnet und geschlossen wird, und ein bewegtes Bild (iib) des Gesichts, bei dem nur ein Mund geöffnet und geschlossen wird, sind.

7. Vorrichtung zur Unterstützung der Diagnose von Autismus nach Anspruch 6,
wobei die Häufigkeit der Bewegung des Blickes des Auges auf der Grundlage eines Kontrasts oder Unterschieds zwischen dem typischen sich entwickelnden Individuum und dem Individuum mit Autismus in Bezug auf eine Tendenz einer Bewegung des Blickes des Auges ausgewertet wird, wobei, während das bewegte Bild (iia) des Gesichts, bei dem nur das Auge geöffnet und geschlossen wird, angezeigt wird, die Häufigkeit der Bewegung des Blickes des Auges des typischen sich entwickelnden Individuums zu einer Peripherie des Auges hoch, die des Individuums mit Autismus aber niedrig ist.

8. Vorrichtung zur Unterstützung der Diagnose von Autismus nach Anspruch 6,
wobei die Häufigkeit der Bewegung des Blickes des Auges auf der Grundlage eines Kontrasts oder Unterschieds zwischen dem typischen sich entwickelnden Individuum und dem Individuum mit Autismus in Bezug auf eine Tendenz einer Bewegung des Blickes des Auges ausgewertet wird, wobei die Häufigkeit der Bewegung des Blickes des Auges des Individuums mit Autismus nach (iia), in einem Fall, in dem zuerst das bewegte Bild (iib) des Gesichtes, bei dem nur der Mund geöffnet und geschlossen wird, und dann das bewegte Bild (iia) des Gesichtes, bei dem nur das Auge geöffnet und geschlossen wird, angezeigt wird, im Vergleich zu dem des typischen sich entwickelnden Individuums, in einem Fall, in dem das Standbild (ia) des Gesichts oder das bewegte Bild (iib) des Gesichts, bei dem nur der Mund geöffnet und geschlossen wird, angezeigt wird, gering ist.

9. Vorrichtung zur Unterstützung der Diagnose von Autismus nach einem der Ansprüche 1 bis 8,
wobei ein Bild einer Person, die das Subjekt kennt, als das vordefinierte menschliche Bild (I) verwendet wird.

10. Vorrichtung zur Unterstützung der Diagnose von Autismus nach Anspruch 1,
wobei das vorbestimmte nicht-menschliche Bild (II) das Erscheinungsvorhersagebild (α)enthält, und
wobei der Teilbereich zum Auswerten der Informationen über die Position des Blickes des Auges eine Häufigkeit einer Bewegung des Blickes des Auges zu einem Zeitpunkt der erneuten Anzeige des Bilds (α1) des sich bewegenden Körpers an einer vorbestimmten Position in dem Anzeigeabschnitt (a3) auswertet, nachdem das Bild (α1) des sich bewegenden Körpers zuerst in einer solchen Weise angezeigt wird, dass es sich auf einem Bildschirm auf dem Anzeigeabschnitt (a3) bewegt, und dann das Bild (α1) des sich bewegenden Körpers in einen nicht angezeigten Zustand überführt wird, indem es sich außerhalb des Bildschirms des Anzeigeabschnitts (a3) befindet oder durch das Bild (α2) des sich verdeckenden Körpers, auf der Grundlage eines Kontrasts oder Unterschieds zwischen dem typischen sich entwickelnden Individuum und dem Individuum mit Autismus in Bezug auf eine Tendenz der Bewegung des Blickes des Auges, wobei die Häufigkeit der Bewegung des Blickes des Auges des typischen sich entwickelnden Individuums in eine Position, in der das Bild (α1) des sich bewegenden Körpers erneut angezeigt wird hoch, die des Individuums mit Autismus jedoch niedrig ist.

11. Vorrichtung zur Unterstützung der Diagnose von Autismus nach Anspruch 10,
wobei der Teilbereich zum Auswerten der Informationen über die Position des Blickes des Auges nicht die Häufigkeit der Bewegung des Blickes des Auges zum Zeitpunkt der erneuten Anzeige für die Auswertung der Häufigkeit der Bewegung verwendet, wenn die erneute Anzeige zum ersten Mal implementiert wird, sondern für die Auswertung der Häufigkeit der Bewegung verwendet, wenn die erneute Anzeige zum zweiten Mal oder danach implementiert wird, wobei eine Bewegung eines Bildes (α1) eines sich bewegenden Körpers unter einer bestimmten Regel vorhersagbar ist.

12. Vorrichtung zur Unterstützung der Diagnose von Autismus nach Anspruch 1, wobei das vorbestimmte nicht-menschliche Bild (II) das Illusionserkennungsbild (β) enthält, und
wobei der Teilbereich zum Auswerten der Informationen über die Position des Blickes des Auges eine Häufigkeit einer Bewegung des Blickes des Auges, in einem Fall, in dem das die Illusion verursachende Element (β1) angezeigt wird, auswertet, auf der Grundlage eines Kontrasts oder Unterschieds zwischen dem typischen sich entwickelnden Individuum und dem Individuum mit Autismus in Bezug auf eine Tendenz der Bewegung des Blickes des Auges, wobei die Häufigkeit der Bewegung des Blickes des Auges des typischen sich entwickelnden Individuums zwischen einer Position, bei der das die Illusion verursachende Element (β1) angezeigt wird, und einer Position, bei der das keine Illusion verursachende Element (β2) angezeigt wird, hoch, die des Individuums mit Autismus aber niedrig ist.

13. Vorrichtung zur Unterstützung der Diagnose von Autismus nach Anspruch 1,
wobei das vorbestimmte nicht-menschliche Bild (II) das Differenzsuchbild (γ) enthält, und
wobei der Teilbereich zum Auswerten der Informationen über die Position des Blickes des Auges eine Häufigkeit einer Bewegung des Blickes des Auges, in einem Fall, in dem die identischen Bilder (γ1) und die unterschiedlichen Bilder (γ2) in gemischter Weise auf dem auf dem Anzeigeabschnitt (a3) angezeigt werden, auswertet, auf der Grundlage eines Kontrasts oder Unterschieds zwischen dem typischen sich entwickelnden Individuum und dem Individuum mit Autismus in Bezug auf eine Tendenz einer Bewegung des Blickes des Auges, wobei die Häufigkeit der Bewegung des Blickes des Auges des typischen sich entwickelnden Individuums zwischen einer Position, bei der das identische Bild (γ1) angezeigt wird, und einer Position, bei der unterschiedliche Bild (γ2) angezeigt wird, niedrig, die des Individuums mit Autismus aber hoch ist.

14. Vorrichtung zur Unterstützung der Diagnose von Autismus nach Anspruch 1,
wobei, bevor das Kombinationsbild auf einem Bildschirm des Anzeigeabschnitts (a3) angezeigt wird, ein vorläufiges Bildführungsbild (θ) auf dem Bildschirm angezeigt wird, um den Blick des Auges des Subjekts im Voraus zu einer vorbestimmten Position zu führen.

## Revendications

1. Dispositif d'assistance au diagnostic de l'autisme assistant le diagnostic de l'autisme, le dispositif comprenant:
(i) une partie de détection du regard propre à afficher, sur une partie d'affichage (a3) à disposer sur une position dans la direction du regard d'un sujet, une combinaison d'images pour afficher séquentiellement au moins deux images comprenant une image humaine prédéterminée (I) et une image non humaine prédéterminée (II), et propre à détecter les informations sur la position du regard sur le sujet regardant la combinaison d'images;
(ii) une partie stockant les informations sur la position du regard propre à stocker les informations sur la position du regard détectées par la partie de détection du regard;
(iii) une partie d'affichage des informations sur la position du regard propre à afficher les informations sur la position du regard sur le sujet stockées dans la partie de stockage des informations sur la position du regard;
(iv) une partie d'évaluation des informations sur la position du regard propre à évaluer les informations sur la position du regard sur le sujet en comparant les informations sur la position du regard sur le sujet affichées sur la partie d'affichage des informations sur la position du regard avec les informations sur la position du regard sur l'individu présentant de l'autisme et/ou l'individu au développement typique;
(v) une partie de sortie des résultats d'évaluation propre à sortir des résultats d'évaluation obtenus par la partie d'évaluation des informations sur la position du regard; et
(vi) une partie de stockage des résultats d'évaluation propre à stocker les résultats d'évaluation sortis de la partie de sortie des résultats d'évaluation ou des résultats d'évaluation obtenus par la partie d'évaluation des informations sur la position du regard,
dans lequel l'image non humaine prédéterminée (II) comprend au moins un type sélectionné parmi une image à prédiction de l'apparence (α), une image de reconnaissance d'une illusion (β) et une image de recherche de la différence (γ), dans lequel l'image à prédiction de l'apparence (α) est une image animée comprenant une image de corps animée (α1) qui est une figure animée sur la partie d'affichage (a3) soumise à une certaine règle, dans lequel l'image animée ne s'affiche pas pendant une période de temps prédéterminée, quand le corps animé se déplace hors de l'écran ou quand une image de corps cachée (α2), qui est une autre figure affichée sur l'écran, chevauche l'image de corps animée (α1), de sorte qu'il semble que le corps animé soit disposé derrière l'image de corps cachée (a2), puis réapparaît sur l'écran,
dans lequel l'image de reconnaissance d'une illusion (β) est une image formée de figures comprenant un élément causant l'illusion (β1) et un élément causant la non illusion (β2) affichées sur l'écran de la partie d'affichage (a3), et
dans lequel l'image de recherche de la différence (γ) est une image formée d'une combinaison d'une pluralité d'images identiques (γ1) présentant la même apparence ou une semblable, et une ou plusieurs figures différentes (γ2) ayant une forme différente de celles des figures identiques, et les figures identiques (γ1) et les figures différentes (γ2) s'affichent d'une manière mélangée sur la partie d'affichage (a3).

2. Dispositif d'assistance au diagnostic de l'autisme selon la revendication 1,
dans lequel l'image non humaine prédéterminée (II) comprend une image à prédiction de l'apparence (α), une image de reconnaissance d'une illusion (β) et une image de recherche de la différence (γ).

3. Dispositif d'assistance au diagnostic de l'autisme selon l'une quelconque de la revendication 1 ou 2,
dans lequel la partie de détection du regard est propre à détecter les informations sur la position du regard sur le sujet regardant un écran de la partie d'affichage (a3), en utilisant une partie de caméra (a1) capturant l'image de l'œil du sujet, ou une partie d'électrode (a2) à assembler sur la tête du sujet et détecter un mouvement de l'œil du sujet.

4. Dispositif d'assistance au diagnostic de l'autisme selon l'une quelconque des revendications 1 à 3,
dans lequel la partie sortant les résultats d'évaluation est propre à afficher les résultats d'évaluation de la position du regard du sujet.

5. Dispositif d'assistance au diagnostic de l'autisme selon l'une quelconque des revendications 1 à 4,
dans lequel l'image humaine prédéterminée (I) comprend une image fixe (i) et une image animée (ii) en animation partielle, et
dans lequel la partie d'évaluation des informations sur la position du regard évalue la fréquence d'un mouvement du regard, dans le cas où l'image fixe (i) ou l'image animée (ii) en animation partielle s'affiche sur l'écran de la partie d'affichage (a3), sur base d'un contraste ou d'une différence entre l'individuel au développement typique et l'individu présentant de l'autisme en termes de tendance d'un mouvement du regard où la fréquence du mouvement du regard de l'individu au développement typique vers la partie animée de l'image animée est élevée, mais celle de l'individu présentant de l'autisme est faible.

6. Dispositif d'assistance au diagnostic de l'autisme selon la revendication 5,
dans lequel l'image humaine prédéterminée (I) comprend trois types d'images, lesquelles sont l'image fixe (ia) d'un visage, l'image animée (iia) du visage où seul un œil est ouvert puis fermé, et l'image animée (iib) du visage où seule la bouche est ouverte puis fermée.

7. Dispositif d'assistance au diagnostic de l'autisme selon la revendication 6,
dans lequel la fréquence du mouvement du regard est évaluée sur base d'un contraste ou d'une différence entre l'individu au développement typique et l'individu présentant de l'autisme en termes de tendance du mouvement du regard où, pendant l'affichage de l'image animée (iia) du visage où seul un œil est ouvert puis fermé, la fréquence du mouvement du regard de l'individu au développement typique vers la périphérie de l'œil est élevée, mais celle de l'individu présentant de l'autisme est faible.

8. Dispositif d'assistance au diagnostic de l'autisme selon la revendication 6,
dans lequel la fréquence du mouvement du regard est évaluée sur base d'un contraste ou d'une différence entre l'individu au développement typique et l'individu présentant de l'autisme en termes de tendance du mouvement du regard, où la fréquence du mouvement du regard de l'individu présentant de l'autisme vers (iia) dans le cas où l'image animée (iib) du visage où seule la bouche est ouverte puis fermée, s'affiche d'abord puis l'image animée (iia) du visage où seul l'œil est ouvert puis fermé s'affiche est faible, comparée à celle de l'individu au développement typique dans le cas où l'image fixe (ia) du visage ou l'image animée (iib) du visage où seule la bouche est ouverte puis fermée s'affiche.

9. Dispositif d'assistance au diagnostic de l'autisme selon l'une quelconque des revendications 1 à 8, dans lequel l'image d'une personne que le sujet connaît, est utilisée comme image humaine prédéterminée (I).

10. Dispositif d'assistance au diagnostic de l'autisme selon la revendication 1,
dans lequel l'image non humaine prédéterminée (II) comprend l'image à prédiction de l'apparence (α), et
dans lequel la partie d'évaluation des informations sur la position du regard évalue la fréquence du mouvement du regard à un moment de réaffichage de l'image de corps animée (α1) à une position prédéterminée dans la partie d'affichage (a3), après premier affichage de l'image de corps animée (α1) d'une manière telle qu'elle se déplace sur un écran sur la partie d'affichage (a3), puis faire que l'image de corps animée (α1) effectue une transition en un état non affiché en disparaissant de l'écran de la partie d'affichage (a3) ou par l'image de corps cachée (a2), sur base d'un contraste ou d'une différence entre l'individuel au développement typique et l'individu présentant de l'autisme en termes de tendance d'un mouvement du regard où la fréquence du mouvement du regard de l'individu au développement typique vers une position où l'image de corps animée (α1) est réaffichée, est élevée, mais celle de l'individu présentant de l'autisme est faible.

11. Dispositif d'assistance au diagnostic de l'autisme selon la revendication 10,
dans lequel la partie d'évaluation des informations sur la position du regard n'utilise pas la fréquence du mouvement du regard au moment du réaffichage pour l'évaluation de la fréquence du mouvement quand le réaffichage est réalisé pour une première fois, mais l'utilise pour l'évaluation de la fréquence du mouvement quand le réaffichage est réalisé une deuxième fois ou après, quand un mouvement, en respectant une certaine règle, d'une image de corps animée (α1) est prévisible.

12. Dispositif d'assistance au diagnostic de l'autisme selon la revendication 1,
dans lequel l'image non humaine prédéterminée (II) comprend l'image de reconnaissance d'une illusion (β), et
dans lequel la partie d'évaluation des informations sur la position du regard évalue la fréquence d'un mouvement du regard, dans le cas où l'élément causant l'illusion (β1) s'affiche, sur base d'un contraste ou d'une différence entre l'individuel au développement typique et l'individu présentant de l'autisme en termes de tendance d'un mouvement du regard où la fréquence du mouvement du regard de l'individu au développement typique entre une position où l'élément causant l'illusion (β1) s'affiche et une position où l'élément causant la non-illusion (β2) s'affiche est élevée, mais celle de l'individu présentant de l'autisme est faible.

13. Dispositif d'assistance au diagnostic de l'autisme selon la revendication 1,
dans lequel l'image non humaine prédéterminée (II) comprend l'image de recherche de la différence (γ), et
dans lequel la partie d'évaluation des informations sur la position du regard évalue la fréquence du mouvement du regard, dans le cas où les figures identiques (γ1) et les figures différentes (γ2) s'affichent d'une manière mélangée sur la partie d'affichage (a3), sur base d'un contraste ou d'une différence entre l'individuel au développement typique et l'individu présentant de l'autisme en termes de tendance d'un mouvement du regard où la fréquence du mouvement du regard de l'individu au développement typique entre une position où la figure identique (γ1) s'affiche, et une position où la figure différente (γ2) s'affiche est faible, mais celle de l'individu présentant de l'autisme est élevée.

14. Dispositif d'assistance au diagnostic de l'autisme selon la revendication 1,
dans lequel la combinaison d'images s'affiche sur un écran de la partie d'affichage (a3), une image principale de l'image préliminaire (θ) s'affiche sur l'écran pour conduire à l'avance le regard du sujet vers une position prédéterminée.
